(19)

**Europäisches Patentamt**
**European Patent Office**
**Office européen des brevets**

(11)  **EP 3 786 962 B1**

(12)  # EUROPEAN PATENT SPECIFICATION

(45) Date of publication and mention
of the grant of the patent:
**17.09.2025  Bulletin 2025/38**

(21) Application number: **20186478.2**

(22) Date of filing: **17.07.2020**

(51) International Patent Classification (IPC):
***G16C 20/30*** *(2019.01)*

(52) Cooperative Patent Classification (CPC):
**G16C 20/30**

(54) **SYSTEMS AND METHODS FOR GENERATING OPTIMIZED SET OF PHARMACOKINETIC (PK) AND PHARMACODYNAMIC (PD) PARAMETERS**

SYSTEME UND VERFAHREN ZUR ERZEUGUNG EINES OPTIMIERTEN SATZES VON PHARMAKOKINETISCHEN (PK) UND PHARMAKODYNAMISCHEN (PD) PARAMETERN

SYSTÈMES ET PROCÉDÉS PERMETTANT DE GÉNÉRER UN ENSEMBLE OPTIMISÉ DE PARAMÈTRES PHARMACOCINÉTIQUES (PK) ET PHARMACODYNAMIQUES (PD)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.07.2019  IN 201921029215**

(43) Date of publication of application:
**03.03.2021  Bulletin 2021/09**

(73) Proprietor: **Tata Consultancy Services Limited Maharashtra (IN)**

(72) Inventors:
• **RAMAMURTHI, Narayanan**
  **500081 Hyderabad, Telangana (IN)**
• **DAS, Shyam Sundar**
  **500081 Hyderabad, Telangana (IN)**
• **KONETI, Geervani**
  **500081 Hyderabad, Telangana (IN)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
**EP-A1- 3 422 358          WO-A1-2016/000035**
**KR-A- 20110 119 409      US-A1- 2003 088 320**
**US-A1- 2015 269 225**

• **ABDULHAMID S. M. ET AL: "A Survey of League Championship Algorithm: Prospects and Challenges", vol. 8, no. S3, 1 February 2015 (2015-02-01), IN, pages 101 - 110, XP055766398, ISSN: 0974-6846, Retrieved from the Internet <URL:https://arxiv.org/ftp/arxiv/papers/1603/ 1603.09728.pdf> [retrieved on 20210118], DOI: 10.17485/ijst/2015/v8iS3/60476**

**Description**

CROSS-REFERENCE TO RELATED APPLICATIONS AND PRIORITY

The present application claims priority to Indian complete specification titled "SYSTEMS AND METHODS FOR GENERATING OPTIMIZED SET OF PHARMACOKINETIC (PK) AND PHARMACODYNAMIC (PD) PARAMETERS", Application No. 201921029215, filed in India on July 19, 2019.

TECHNICAL FIELD

The disclosure herein generally relates to optimization techniques, and, more particularly, to systems and methods for generating an optimized set of Pharmacokinetic (PK) and Pharmacodynamic (PD) parameters from appropriate animal, human or simulated data using known PK-PD models and Hybrid Modified League Championship Algorithm (HMLCA) method.

BACKGROUND

Developability of a drug candidate is decided based on the Pharmacokinetic (PK) and Pharmacodynamic (PD) parameters of the drug candidate under investigation, usually estimated from Plasma Concentration Time (PCT) and response time profiles of the drug candidate, measured in a number of targeted animal/Human in vivo studies. PK is the study of what the body does to a drug, i.e., its absorption, distribution, metabolism and excretion profiles. On the other hand, PD defines what a drug does to the body. PD modeling attempts to characterize measured physiological parameters before and after drug administration with the effect defined as the change in parameter relative to pre-dose or baseline value.

The objective of these models is to estimate the PK/PD parameters which is an integral part of model based drug development. The significance of accurately estimating these parameters lies in the fact that many of these parameters cannot be measured experimentally either in human or in animal. Further, the decision to take the compound/drug to the next level of drug development process depends heavily on the accuracy of the parameters values. The accuracy of the optimized parameter estimates obtained using available optimization methods such as Gauss-Newton etc., is dependent on appropriate selection of initial parameter values. These estimations may be an educated guess based on the structure of the model, or may be determined by using estimated parameters from previous studies or based on non-compartmental analysis or curve striping, grid search etc. approaches. However, these approaches do not always guarantee good initial estimates of all the PK-PD parameters of interest. Further, the number of parameters and complexity of the model increase the execution time required for a computing method to estimate the parameters. Further, usually, grid search (GS) method with a fixed number of grid points has been used to calculate initial values using parameter bounds. Though it is a simple and deterministic method, it is highly dependent on the parameter bounds. If the user provides a wider parameter bounds, then GS has to use a higher number of grid points which is not possible if the grid point number is fixed. Furthermore, increase in grid point number exponentially increases the execution time and thus making them less efficient for PK-PD data analysis.

US 2015/269225 A1 discloses a system and method for parallelizing grid search technique facilitating determination of PK-PD parameters. The method may comprise determining number of grids. The method may further comprise creating grid points based upon the number of grids (N) and a number of parameters (p). The method may further comprise distributing the grid points amongst number of threads. The method may further comprise evaluating an objective function value corresponding to each grid point in order to compute an objective function value associated with each of the grid points. Further, the method may comprise identifying a grid point having minimum objective function value. The grid point having the least objective function value may indicate the estimated initial PK-PD parameters.

EP 3 422 358 A1 discloses a method for predicting a value of a parameter of a system (20). The value of the parameter of the system (20) is predicted by incrementally optimizing a pharmacokinetic and pharmacodynamic model based on values of parameters of the system (20) received overtime. This allows for predicting values of the parameters of the system (20) with improved accuracy. In one embodiment the system (20) comprises a coagulation system (21) comprising an anticoagulant. The predicted value of the parameter of the system (20) can be a point in time at which the coagulation system (21) reaches hemostatic balance after a periodic supply of anticoagulant to the coagulation system (21) is discontinued.

KR 20110119409 A discloses integration analysis system for the stir - pharmacodynamic modeling of the drug, providing the stir - pharmacodynamic modeling method of the drug determining the optimized pharmacokinetics population parameter comprising the stir - pharmacodynamic modeling of the drug more specifically injected to the patient.

WO 2016/000035 A1 discloses a method of modelling system behaviour of a physical system, the method including, in one or more electronic processing devices obtaining quantified system data measured for the physical system, the quantified system data being at least partially indicative of the system behaviour for at least a time period, forming at least one population of model units, each model unit including model parameters and at least part of a model, the model parameters being at least partially based on the quantified system data, each model including one or more mathematical equations for modelling system behaviour, for each model unit calculating at least one solution trajectory for at least part of the at least one time period; determining a fitness value based at least in part on the at least one solution trajectory; and, selecting a combination of model units using the fitness values of each model unit, the combination of model units representing a collective model that models the system behaviour.

US 2003/088320 A1 discloses an automated method for identifying an optimal or near optimal mathematical model to describe observed data including: a) the definition of a candidate model search space, b) methods for searching said candidate model search space to identify the optimal or near optimal model within said candidate model search space. The present invention includes algorithms for writing the computer code needed to implement and evaluate candidate models in the software package NONMEM.

"A Survey of League Championship Algorithm: Prospects and Challenges" discloses the League Championship Algorithm (LCA) Js spart-inspired optimization algorithm that was introduced by A1 Husseinzadeh Kashan in the year 2009. It has since drawn enormous interest among the researchers because of its potential efficiency in solving many optimization problems and real-world applications. The LCA has also shown great potentials in salving nondeterministic polynomial time (NP-complete) problems. This survey presents a brief synopsis of the LCA literatures in peer-reviewed journals, conferences and book chapters. These research articles are then categorized according to indexing in the major academic databases (Web Science, Scopus, IEEE Xplore and the Google Scholar).

## SUMMARY OF THE INVENTION

The invention is defined in the appended claims, in particular independent claims.

## SUMMARY OF RELATED DISCLOSURE

Embodiments of the present disclosure present technological improvements as solutions to the above-mentioned technical problems recognized by the inventors in conventional systems. For example, in one aspect, there is processor implemented method for generating optimized PK-PD parameter values. In one aspect, there is provided a processor implemented method for generating optimized set of Pharmacokinetic (PK) and Pharmacodynamic (PD) parameters. The method comprises obtaining, via one or more hardware processors, using a Hybrid Modified League Championship Algorithm, HMLCA, a set of data pertaining to one of a Pharmacokinetics (PK) model or a Pharmacodynamics (PD) model, dosage information associated with the set thereof, a concentration-time or a response-time data, and a parameter boundary for the PK model or the PD model, wherein the set of data comprises parameters values corresponding to one of PK parameters or PD parameters; randomly generating, using the HMLCA, a set of potential solutions as a population based on the parameter boundary and assigning generated parameters values as a current best formation for each potential solution from the set, wherein each potential solution from the population comprises parameter values that are within the parameter boundary; computing, using the HMLCA, a fitness value for each potential solution from the population based on one or more criteria, wherein, the fitness value is calculated in the form of an objective function, and wherein the one or more criteria comprises at least one of strength and weakness pertaining to each of the plurality of potential solutions. The method further comprises computing, using the HMLCA, a global optimal solution among the potential solutions from the population, wherein the global optimal solution comprises optimal parameter values from initialized parameter values; pairing, using the HMLCA, until a stopping criteria is satisfied, the potential solutions across each other based on a predefined rule to obtain unique pairs of potential solutions, wherein the unique pairs of potential solutions are being identified for (i) comparison against each other and (ii) subsequent updation of each potential solution, wherein the stopping criteria is defined that is indicative of number of times each solution needs an update; determining, using the HMLCA, using a fitness value associated with (i) each potential solution from the unique pairs of potential solutions and (ii) the global optimal solution, a type I potential solution and type II potential solution from each of the unique pairs; optimizing, using the HMLCA, the fitness value of the type I potential solution and type II potential solution by performing a crossover of one or more parameter values associated thereof; generating, using the HMLCA, new potential solutions based on the current optimal parameter value of the type I potential solution and type II potential solution; performing, using the HMLCA, a comparison of a fitness value of the parameter values corresponding to the new solutions with a fitness value of (i) current optimal parameter values of the new solutions and (ii) the global optimal solution and updating, based on the comparison, the current optimal parameter values with the parameter values for each new solution and the global optimal solution; eliminating, using the HMLCA, a subset of the new solutions based on a comparison of a fitness value of each of the new solutions with a fitness threshold to obtain a filtered set of solutions, wherein each solution comprised in the filtered set of solutions includes optimized parameter values, wherein each of the optimized parameter values comprises a fitness value that is less than or equal to the fitness threshold; adding, using the HMLCA, a new set of randomly generated potential solutions into the filtered set of solutions to obtain an updated population; generating, using the HMLCA, a global optimal solution from the updated population based on an optimal fitness value; and performing, using the HMLCA, a local optimization technique on the global optimal solution to estimate a set of optimized parameter values.

In another aspect, there is provided a processor implemented system for generating optimized set of Pharmacokinetic (PK) and Pharmacodynamic (PD) parameters. The system comprises a memory storing instructions; one or more communication interfaces; and one or more hardware processors coupled to the memory via the one or more communication interfaces, wherein the one or more hardware processors using a Hybrid Modified League Championship Algorithm, HMLCA, are configured by the instructions to: obtain a set of data pertaining to one of a Pharmacokinetics (PK) model or a Pharmacodynamics (PD) model, dosage information associated with the set thereof, a concentration-time or a response-time data, and a parameter boundary for the PK model or the PD model, wherein the set of data comprises parameters values corresponding to one of PK parameters or PD parameters; randomly generate a set of potential solutions as a population based on the parameter boundary and assign generated parameter values as current best formation for each potential solution from the set, wherein each solution from the set of potential solutions comprises parameter values that are within the parameter boundary; compute a fitness value for each potential solution of the population based on one or more criteria, wherein, the fitness value is calculated in the form of an objective function (e.g., weighted residual sum squares (WRSS)), and wherein the one or more criteria comprises at least one of strength and weakness pertaining to each of the plurality of potential solutions. The one or more hardware processors are further configured by the instructions to compute a global optimal solution among the potential solutions, wherein the global optimal solution comprises optimal parameter values from initialized parameter values; pair, until a stopping criteria is satisfied, the potential solutions across each other based on a predefined rule to obtain unique pairs of potential solutions, wherein the unique pairs of potential solutions are being identified for (i) comparison against each other and (ii) subsequent updation of each potential solution, wherein the stopping criteria is defined that is indicative of number of times each solution needs an update; determine, using a fitness value associated with (i) each potential solution from the unique pairs of potential solutions and (ii) the global optimal solution, a type I potential solution and type II potential solution from each of the unique pairs; optimize the fitness value of the type I potential solution and type II potential solution by performing a crossover of one or more parameter values associated thereof; generate new potential solutions based on the current optimal parameter value of the type I potential solution and type II potential solution; perform a comparison of a fitness value of the parameter values corresponding to the new solutions with a fitness value of (i) current optimal parameter values of the new solutions and (ii) the global optimal solution and update, based on the comparison, the current optimal parameter values with the parameter values for each new solution and the global optimal solution.

The one or more hardware processors are further configured by the instructions to eliminate a subset of the new solutions based on a comparison of a fitness value of each of the new solutions with a fitness threshold to obtain a filtered set of solutions, wherein each solution comprised in the filtered set of solutions includes optimized parameter values, wherein each of the optimized parameter values comprises a fitness value that is less than or equal to the fitness threshold; add a new set of randomly generated potential solutions into the filtered set of solutions to obtain an updated population; generate a global optimal potential solution from the updated population based on an optimal fitness value; and perform a local optimization technique on the global optimal solution to estimate a set of optimized parameter values.

In one aspect, there are provided one or more non-transitory machine readable information storage mediums comprising one or more instructions which when executed by one or more hardware processors using a Hybrid Modified League Championship Algorithm, HMLCA, causes generation of optimized set of Pharmacokinetic (PK) and Pharmacodynamic (PD) parameters by obtaining a set of data pertaining to one of a Pharmacokinetics (PK) model or a Pharmacodynamics (PD) model, dosage information associated with the set thereof, a concentration-time or a response-time data, and a parameter boundary for the PK model or the PD model, wherein the set of data comprises parameters values corresponding to one of PK parameters or PD parameters; randomly generating a set of potential solutions as a population based on the parameter boundary and assigning generated parameter values as a current best formation for each potential solution from the set, wherein each of the potential solutions from the set (or the population) comprises parameter values that are within the parameter boundary; computing a fitness value for each of the potential solutions based on one or more criteria, wherein, the fitness value is calculated in the form of an objective function (e.g., weighted residual sum squares (WRSS)), and wherein the one or more criteria comprises at least one of strength and weakness pertaining to each of the plurality of potential solutions. The method further comprises computing a global optimal solution among the potential solutions/population, wherein the global optimal solution comprises optimal parameter values from initialized parameter values; pairing, until a stopping criteria is satisfied, the potential solutions across each other based on a predefined rule to obtain unique pairs of potential solutions, wherein the unique pairs of potential solutions are being identified for (i) comparison against each other and (ii) subsequent updation of each potential solution, wherein the stopping criteria is defined that is indicative of number of times each solution needs an update; determining, using a fitness value associated with (i) each potential solution from the unique pairs of potential solutions and (ii) the global optimal solution, a type I potential solution and type II potential solution from each of the unique pairs;

optimizing the fitness value of the type I potential solution and type II potential solution by performing a crossover of one or more parameter values associated thereof; generating new potential solutions based on the current optimal parameter value of the type I potential solution and type II potential solution; performing a comparison of a fitness value of the parameter values corresponding to the new solutions with a fitness value of (i) current optimal parameter values of the new solutions and (ii) the global optimal solution and updating, based on the comparison, the current optimal parameter values with the parameter values for each new solution and the global optimal solution; eliminating a subset of the new solutions based on a comparison of a fitness value of each of the new solutions with a fitness threshold to obtain a filtered set of solutions, wherein each solution comprised in the filtered set of solutions includes optimized parameter values, wherein each of the optimized parameter values comprises a fitness value that is less than or equal to the fitness threshold; adding a new set of randomly generated potential solutions into the filtered set of solutions to obtain an updated population; generating a global optimal solution from the updated population based on an optimal fitness value; and performing a local optimization technique on the global optimal solution to estimate a set of optimized parameter values.

This summary is provided to introduce aspects related to systems and methods for optimizing PK-PD parameters in a parameter space and the concepts are further described below in the detailed description. This summary is not intended to identify essential features of subject matter nor is it intended for use in determining or limiting the scope of the subject matter. In other words, above summary is described for better understanding of the embodiments of the present disclosure by way of following description and examples: A set of PCT or response-time data, user defined Pharmacokinetics (PK) model or a Pharmacodynamics (PD) model, dosage information as appropriate to a specific scenario and a parameter boundary for the PK model or the PD model are obtained as input. The proposed two step optimization method Hybrid Modified League Championship Algorithm (HMLCA) as implemented by the present disclosure utilized above go through following process to compute an optimized solution.

It is to be understood that the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 depicts a typical conventional two step parameters optimization.

FIG. 2 illustrates an exemplary block diagram of a system for estimating of Pharmacokinetic-Pharmacodynamic (PK-PD) Parameters using Hybrid Modified League Championship Algorithm in accordance with an embodiment of the present disclosure.

FIGS. 3A-3B illustrate an exemplary flow diagram of a method for estimating of Pharmacokinetic-Pharmacodynamic (PK-PD) Parameters using the Hybrid Modified League Championship Algorithm executed by the system of FIG. 2 in accordance with an embodiment of the present disclosure.

FIG. 4 depicts a graphical representation of Plasma Concentration Time (PCT) profile of the administered doses in accordance with an example embodiment of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Estimating PK/PD parameters is an integral part of model based drug development. The significance of accurately estimating these parameters lies in the fact that many of these parameters cannot be measured experimentally either in human or in animal. Further, the decision to take the compound/drug to the next level of drug development process depends heavily on the accuracy of the parameters values. The accuracy of the optimized parameter estimates obtained using available computing methods such as Gauss-Newton etc., is dependent on appropriate selection of initial parameter values. These estimations may be an educated guess based on the structure of the model, or may be determined by using estimated parameters from previous studies. However, these approaches do not always guarantee good initial estimates of all the PK-PD parameters of interest.

All commercial and public tool, softwares or methods require at least one of the a) initial parameter values and b) parameter bounds for estimating optimal PK/PD parameters. The success of these methods, defined in terms of convergence, optimality, speed, accuracy, memory consumption, resource utilization and others, depends on the quality of both the initial parameter values and the parameter bounds. However, estimation of reasonable initial parameter values, by the user, that assist in convergence of the optimization method is not a straight forward task. Providing reasonable initial parameter values requires comprehensive knowledge of pharmacokinetics and pharmacodynamics, which may be scarce and is one of the bottlenecks for PK/PD parameter estimation. Further, most of the PK/PD optimization methods in commercial tools fail to obtain optimal parameters due to convergence issues or need greater execution times and memory to obtain optimal estimates. Hence, a good initial estimate of parameters for PK/PD parameter optimization becomes a critical component for success of optimization process. Embodiments of the present disclosure provide systems and methods for PK-PD parameter optimization process that ensure a reasonably good initial estimate is obtained for PK-PD optimization process, addressing the bottlenecks discussed above.

Two step optimization process has been introduced previously in literature and tools to address the dependency on initial parameter values. This process consists of two stages Stage 1: initial parameters (IP) are computed using an initial parameter estimation method and user provided parameter bounds and Stage 2: the IP estimated in stage 1 are then optimized using an another set of optimization methods, more specially named local optimization techniques. Some of the estimation methods used in stage 1 are curve stripping, grid search, etc. The drawbacks of these methods used in stage one are a) use of considerable time and memory to obtain reasonable IP estimates, b) restricted scope and scale of applicability and others. The present disclosure is designed to address aforementioned issues of the PK-PD parameter estimation process which will make use of newly designed hybrid modified league championship algorithm (HMLCA) to produce robust and optimal parameter values.

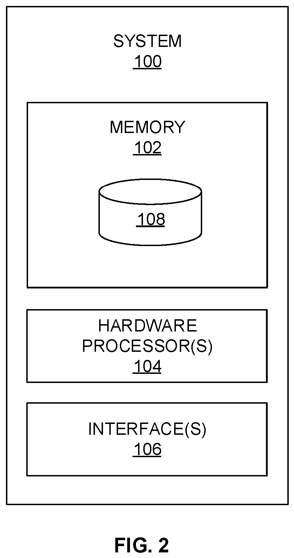

Referring now to the drawings, and more particularly to FIGS. 2 through 4, where similar reference characters denote corresponding features consistently throughout the figures, there are shown preferred embodiments and these embodiments are described in the context of the following exemplary system and/or method.

FIG. 2 illustrates an exemplary block diagram of a system 100 for estimation of Pharmacokinetic-Pharmacodynamic (PK-PD) Parameters using Hybrid Modified League Championship Algorithm in accordance with an embodiment of the present disclosure. The system 100 may also be referred as 'an optimization system' or 'HMLCA system' and interchangeably used hereinafter. In an embodiment, the system 100 includes one or more processors 104, communication interface device(s) or input/output (I/O) interface(s) 106, and one or more data storage devices or memory 102 operatively coupled to the one or more processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is configured to fetch and execute computer-readable instructions stored in the memory. In an embodiment, the device 100 can be implemented in a variety of computing systems, such as laptop computers, notebooks, hand-held devices, workstations, mainframe computers, servers, a network cloud and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment a database 108 can be stored in the memory 102, wherein the database 108 may comprise, but are not limited to information pertaining to Pharmacokinetics (PK) model, a Pharmacodynamics (PD) model, dosage information associated with a set thereof, and a parameter boundary for the PK model and the PD model, wherein the set of data comprising one or more parameter values corresponding to PK parameters and PD parameters, and the like. In an embodiment, the memory 102 may store one or more technique(s) (e.g., local optimization technique(s), global optimization technique(s), and the like) which when executed by the one or more hardware processors 104 to perform the methodology described herein.

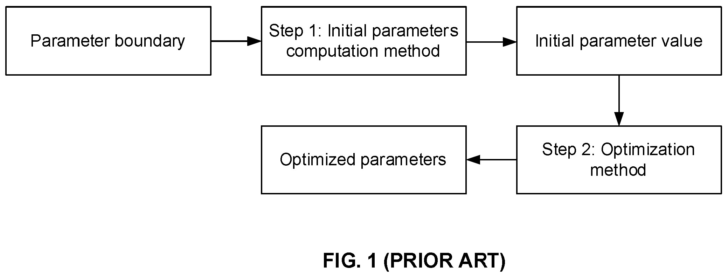

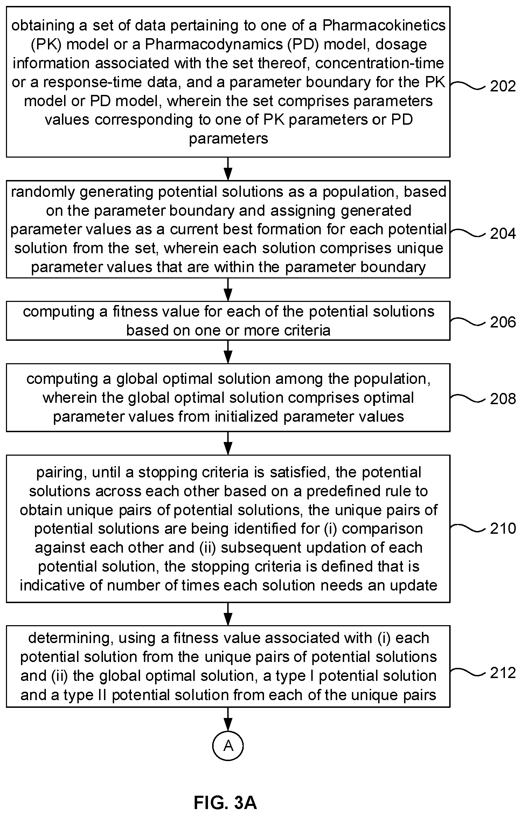

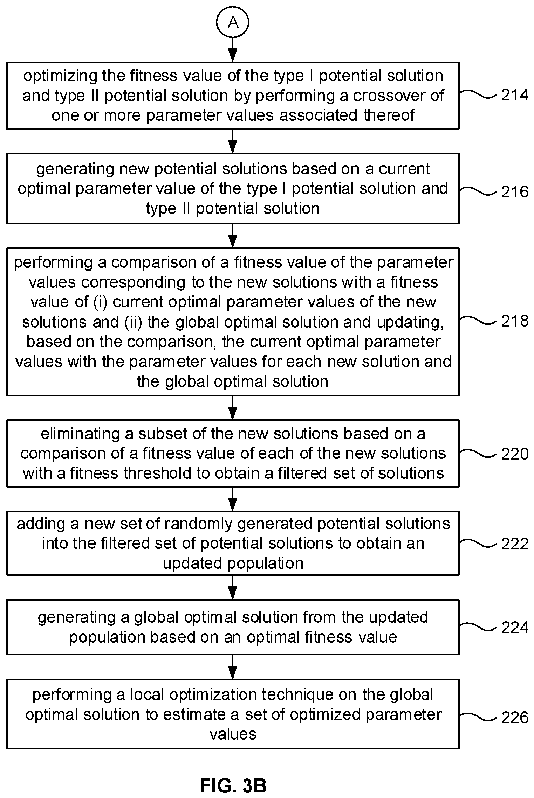

FIGS. 3A-3B, with reference to FIG. 1, illustrate an exemplary flow diagram of a method for estimating of Pharmacokinetic-Pharmacodynamic (PK-PD) Parameters using the Hybrid Modified League Championship Algorithm executed by the system 100 of FIG. 1 in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to the components of the system 100 as depicted in FIG. 2, the flow diagram (FIG. 3A, FIG. 3B) and a case study for which improved performance has been achieved by proposed method.

Referring to FIG. 3A, at step 202 of the present disclosure, the one or more hardware processors 104 obtain a set of data pertaining to one of a) a Pharmacokinetics (PK) model or a Pharmacodynamics (PD) model, b) dosage information associated with the set thereof, c) a concentration-time or a response-time data, and d) a parameter boundary for the PK model or the PD model parameters. In other words, the set of data can be obtained either for PK model or PD model. The PK or PD model consists of a set of equations governing the concentration-time or a response-time data or profiles accordingly. For example the below equation (1) depicts a one compartmental model with intravenous bolus dose of $D_{iv}$. This model consists of two parameters V and $K_{10}$ and is completely described by optimized values of these parameters.

$$C = \frac{D_{iv}}{V} \times e^{-K_{10} \cdot t} \dots \text{ equation } (1)$$

In an embodiment, the set of data comprises parameter values corresponding to one of PK model and parameters or PD model and parameters. In other words, if a set of data pertaining to PK model is obtained, associated dosage information is also obtained accordingly. Likewise, parameter boundary for the PK model is also obtained. The set of data in this case comprises parameter values pertaining to PK parameters. Similarly, in other words, if a set of data pertaining to PD model is obtained, associated dosage information is also obtained accordingly. Likewise, parameter boundary for the PD model is also obtained. The set of data in this case comprises parameter values pertaining to PD parameters.

In the present disclosure, parameter boundaries may be provided by user(s). The parameter boundary consists of range of values, between lower bound (LB) and upper bound (UB), the PK or PD parameters are allowed to take for a given set of data. Below is an exemplary table (Table 1) comprising information pertaining to parameter, and boundary limits (e.g., lower bound, and upper bound):

*Table 1: Exemplary Parameter Bounds*

| Parameter | Lower Bound (LB) | Upper bound (UB) |
|:---:|:---:|:---:|
| a | 0 | 0.5 |
| b | 0 | 2 |
| c | 0 | 25 |
| d | 0 | 2 |
| e | 0 | 25 |
| g | 0 | 0.08 |

In an exemplary scenario, parameter 'a' is allowed to take values between 0 and 0.5 only. The system 100 may also impose additional restrictions on the value of these parameters such as do not allow negative values, etc.

The system 100 is configured to find a best (or optimal) solution within the defined boundary for each parameter of the model. For example, an optimal solution for a model with three parameters can be depicted as $X^{optimal} = [\alpha, \beta, \gamma] = [0.01, 9.8, 1.4]$, in which each value in the parenthesis corresponds to that particular value of the parameter respectively.

At step 204 of the present disclosure, the one or more hardware processors 104 randomly generate a set of potential solutions, referred as population or league, based on the parameter boundary. The generated parameter values are assigned as a current best formation for each potential solution from the set. Each potential solutions refers to a set of values of all parameters in the considered model i.e., each potential solution a) consists of 'N' number of parameters that describe the PK or PD model in detail, b) comprises parameter values that are within the parameter boundary (e.g., the defined boundary - lower bound and upper bound) c) can be referred as team, current team or team formation interchangeable and d) can be represented as $X_I = [x_{I1}, x_{I2}, ..., x_{IN}]$. For example, a potential solution for a model with two parameters is given as [0.1, 9.1]. The generation of the $i^{th}$ parameter of each potential solution is governed by the below equation (2).

$$Potential\ Solution_I = random * (UB_I - LB_i) + LB_i \quad \text{equation } (2)$$

where a) random is a number between 0 and 1 generated by system 100 independently for each parameter and each potential solution b) $LB_i$ represents the lower bound of $i^{th}$ parameter and c) $UB_i$ represents upper bound of $i^{th}$ parameter. Further, the size of the population or number of potential solutions generated by system 100 is referred to as league size (L) or population size, interchangeably.

At step 206 of the present disclosure, the one or more hardware processors 104 compute a fitness value for each potential solution of the population based on one or more criteria. One or more criteria may comprise strength and weakness of the solution(s) that could also be referred as goodness of fit wherein accuracy of the solution is considered. Objective functions may comprise but are not limited to, Residual Sum of Squares (RSS), Weighted Residual Sum of Squares (WRSS), and Extended Weighted Residual Sum of Squares (EWRSS) (objective function used in extended least squares). Subsequently the system 100 then assigns the initial generated values and the fitness measures of each potential solutions as current best formation of the corresponding potential solution, in the first iteration.

At step 208 of the present disclosure, the one or more hardware processors 104 compute a global optimal solution (also referred as 'global best solution') among the potential solutions/population based on the fitness values or objective functions. For example, $I^{th}$ potential solution of the population or league may have the least residual sum of squares value (objective function/fitness value) and is considered as the best or optimal potential solution or parameter values. In an embodiment, the global optimal solution (or the global best solution) comprises optimal parameter values from initialized set of parameter values. In other words, a global best solution is initialized as the best parameter value among all the solutions.

In an embodiment a stopping criteria can be defined as the number of seasons (s) where, in each season, each of the L potential solutions are scheduled to "play a match" against remaining (L-1) potential solutions, like in a single round robin championship. This process can also be referred as pairing and is executed until a stopping criterion is met. In an example embodiment, this league schedule may be changed or retained during the start of each season. In an embodiment of the present disclosure, at step 210, the one or more hardware processors 104 pair the potential solutions using a predefined rule where all the potential solutions are paired against all other potential solutions exactly once. In other words, pairing of the potential solutions across each other is performed (until a stopping criteria is satisfied) based on a predefined rule to obtain unique pairs of potential solutions, the stopping criteria is defined that is indicative of number of times each solution needs an update. Pairs of potential solutions are being identified for (i) comparison against each other and (ii) subsequent updation of each potential solution. This process can also be referred as generating league schedule.

In an embodiment, system 100 ensures that all the solutions are compared against all other solutions exactly once. Pairings of solutions are decided wherein a comparison with each other is performed at different time intervals of next updation process. In an example embodiment, the total number of unique pairings possible for a league schedule are $L(L - 1)/2$. Following table (Table 2) depicts an exemplary paring of solutions (league schedule) for a population with four potential solutions (A, B, C and D):

*Table 2: Exemplary League Schedule*

| Pairing A & B | Pairing B & C |
|---|---|
| Pairing A & C | Pairing B & D |
| Pairing A & D | Pairing C & D |

At step 212 of the present disclosure, the one or more hardware processors 104 determine a type I potential solution and type II potential solution from each of the unique pairs for the current iteration, using a fitness value associated with (i) each potential solution of the pairs and (ii) the global optimal solution. In other words, the system 100, determines, using a fitness value associated with (i) each potential solution from the unique pairs of potential solutions and (ii) the global optimal solution, a type I potential solution and a type II potential solution from each of the unique pairs. In an embodiment, type I potential solution may be a winner, and type II potential solution may be a loser. The step 212 is preceded by a step of determining whether maximum time interval is reached. Maximum time interval can also be represented as number of weeks in a particular season. In an example embodiment, for a league size of L, there may be $(L - 1)$ weeks in a season. Within each week, $L/2$ number of different pairing of potential solutions may be considered for simultaneous comparison and updation. In total, this may result in $L(L - 1)/2$ unique pairing of potential solutions within each season.

In the present disclosure, at step 212, the one or more hardware processors 104, in a season and a time interval, consider two potential solutions I and J contest in week T with formation of $X_I^T$ and $X_J^T$ and fitness value of $f(X_I^T)$ and $f(X_J^T)$ respectively. Let $f^*$ be the so far found best fitness value. Then $P_I^T$ and $P_J^T$ the probability of I wining the contest and J winning the contest respectively are computed as follows:

$$\frac{f(X_I^T) - f^*}{f(X_J^T) - f^*} = \frac{P_J^T}{P_I^T} \qquad \text{equation (3)}$$

$$P_I^T + P_J^T = 1 \qquad \text{equation (4)}$$

From (3) and (4) the following is obtained:

$$P_I^T = \frac{f(X_J^T) - f^*}{f(X_I^T) + f(X_J^T) - 2*f^*} \quad \text{equation (5)}$$

To simulate win or lose, system 100 generates a random number is between 0 and 1. If this random number is less than or equal to $P_I^T$, then potential solution I wins the contest. Otherwise potential solution J wins.

At step 214 of the present disclosure, the one or more hardware processors 104 optimize the fitness value of the type I potential solution and the type II potential solution by performing a crossover of one or more parameter values of the type I potential solution and the type II potential solution. In other words, parameter values of two solutions (the type I potential solution and the type II potential solution) are exchanged systematically to optimize fitness value or/and accommodate the randomness for each unique pair that has type I potential solution and type II potential solution. The present disclosure introduces crossover to exchange the team formations (solution or parameter values formation) of two potential solutions (or two solutions) to produce better formation. This process addresses the following possible scenarios that may arise during comparison a) a team (or a solution) with overall less strength may have some better part in the formation and b) a team (or a solution) with less playing strength may win a match against a better team and others. Crossover operation ensures exchange of optimal information between two potential solutions, in an iteration, and exploits the information at disposal.

In an embodiment, the system 100 performs crossover only if a less fit solution wins a contest (i.e. a solution with high fitness value) against a better fit solution. Further, system 100 can perform many different crossover operations some of which are defined below.

1. One-Point Crossover: This process is realized by cutting two solutions formations at randomly chosen position (crossing point) and swapping the two tails (refer below representation of a one-point crossover).

*Table 3: Sample One-Point crossover representation*

| Solutions | Before Crossover | After Crossover |
|---|---|---|
| **Solution 1** | A1, B1, C1, $\alpha 1$, $\beta 1$, $\gamma 1$ | A1, B1, C1, $\alpha 1$, $\beta 2$, $\gamma 2$ |
| **Solution 2** | A2, B2, C2, $\alpha 2$, $\beta 2$, $\gamma 2$ | A2, B2, C2, $\alpha 2$, $\beta 1$, $\gamma 1$ |

2. N-Point Crossover: Instead of only one, N breaking points are chosen randomly. Every second section is swapped.
3. Uniform Crossover: For each position, it is decided randomly if the position is swapped.

At step 216 of the present disclosure, the one or more hardware processors 104 generate new potential solutions based on a current optimal parameter value of the type I potential solution and type II potential solution. In a post-contest analysis of team I, if it has won (lost) the game against team J at week T, it is the direct consequence of team strengths (weakness) or it is the direct consequences of weakness (strengths) of team J. Now, let based on the schedule of week T+1, I contest against L. Also assume that L contested against K in the last match. New formations are devised for team I using the following rules:

1. If I was winner and L was winner

$$x_{Id}^{T+1} = b_{Id}^T + y_{Id}^T \left( c_1 r_1 (x_{Id}^T - x_{Kd}^T) + c_1 r_2 (x_{Id}^T - x_{Jd}^T) \right)$$

*for d* = 1,2, ..., N
2. If I was winner and L was loser

$$x_{Id}^{T+1} = b_{Id}^T + y_{Id}^T \left( c_2 r_1 (x_{Kd}^T - x_{Id}^T) + c_1 r_2 (x_{Id}^T - x_{Jd}^T) \right)$$

*for d* = 1,2, ..., N
3. If I was loser and L was winner

$$x_{Id}^{T+1} = b_{Id}^T + y_{Id}^T \left( c_1 r_2 (x_{Id}^T - x_{Kd}^T) + c_2 r_1 (x_{Jd}^T - x_{Id}^T) \right)$$

*for d* = 1,2, ..., N

4. If I was loser and L was loser

$$x_{Id}^{T+1} = b_{Id}^T + y_{Id}^T \left( c_2 r_2 (x_{Kd}^T - x_{Id}^T) + c_2 r_1 (x_{Jd}^T - x_{Id}^T) \right)$$

*for d = 1,2, ... , N*

where d is the dimension index of the formation (parameters), $r_1$ and $r_2$ are uniform random number in [0, 1], $c_1$ and $c_2$ are constant coefficients to scale the contribution of the strengths and weakness components respectively. $y_{Id}^T$ is a binary change variable which indicates whether the d-th element in the current best formation changes or not. $b_{Id}^T$ is the d-th element in the best formation of team I at week T. If the value of $y_{Id}^T$ is 1, then $b_{Id}^T$ will be updated. If the value of $y_{Id}^T$ is 0, then there is no updation. The number of 1 in $y_I^T$ is defined by following equation

$$q_I^T = \frac{\ln(1 - (1 - (1 - p_c)^N)\, r)}{\ln(1 - p_c)}$$

where N is number of parameters, r is a random number that takes a value oin [0,1] and $p_c$ is a control parameter initialized to the user or system defined value at the start of each seasons and is multiplied by "rate of increase of $p_c$", which is also a user or system defined value, at the end of each week.

At step 218 of the present disclosure, the one or more hardware processors 104 perform a comparison of a fitness value of the parameter values corresponding to the new solutions with a fitness value of (i) current optimal parameter values of the new solutions and (ii) the global optimal solution, and based on this comparison the current optimal parameter values and the global optimal solution (e.g., global best solution) are updated with the plurality of the new parameter values.

In a nutshell, fitness value of parameter values of each solution is compared with fitness value of (i) current optimal parameter values of that particular solution and (ii) the global optimal solution and based on this comparison, the current optimal parameter values with the parameter values for each new solution and the global optimal solution are updated accordingly. If the objective function or fitness values of the new solution is better than the current optimal parameter value of the solution, then the current optimal parameter value of the solution is updated to the new parameter value. Also, the global optimal solution is updated if there is any better solution after the updation. Thus, each potential solution and global optimal solution is updated by the system 100 for every week. In one embodiment, the expressions 'best' and 'optimal' may be interchangeably used herein.

At step 220 of the present disclosure, the one or more hardware processors 104 eliminate at least a subset (or subsets) of the new solutions based on a comparison of a fitness value of each of the new solutions with a fitness threshold to obtain a filtered set of solutions (alternatively referred as set of filtered solutions, and interchangeably used hereinafter). Each solution comprised in the filtered set of solutions includes optimized parameter values wherein each of the optimized parameter values comprises a fitness value that is more than, less than or equal to the fitness threshold. The process of eliminating potential solutions that do not meet the objective function threshold criteria helps in avoiding unnecessary computation related to these potential solutions or teams as they may not be improved or their improvement may be slow. In an embodiment the potential solutions can also be eliminated based on a predetermined number or percentage of solutions.

At step 222 of the present disclosure, the one or more hardware processors 104 add a new set of randomly generated potential solutions into the filtered set of potential solutions to obtain an updated population. In other words, K number of randomly generated solution(s) is/were added into the population. Similar to elimination either a fixed number or a predefined percentage of potential solutions can be added into the population. This process helps minimize local optimization by introducing new information.

At step 224 of the present disclosure, the one or more hardware processors 104 generate a global optimal solution from the updated population after the completion of the predefined number of iterations. In other words, the global optimal solution is generated from the updated population based on an optimal fitness value. In the present disclosure, parameters with greatest or least fitness value form an optimal solution that is generated from the updated population.

At step 226 of the present disclosure, the one or more hardware processors 104 perform a local optimization technique on the global optimal solution to estimate a set of optimized parameter values for PK model or PD model which is being obtained in step 202. To increase the precision or to find an optimal solution, local optimization (parameter optimization) can be performed using Gauss Newton, Nelder Mead method and others.

In the present disclosure, the expression 'parameter optimization' refers to process of finding the optimal value of the parameters with the help of method(s) (mentioned above).

Embodiments of the present disclosure provide systems and methods for generating optimized (best) set of parameter values (e.g., PK-PD parameters), particularly, by a) performing crossover technique b) removal of worst solutions c) addition of new solutions and d) applying to PK and PD parameter optimization problem. These new processes introduced in the present disclosure help minimize local optimization, reduce redundant computations or low quality potential solutions, and explore the search region effectively.

The present disclosure can be executed in more than one sequence. In an example embodiment, the system 100 can execute uniform cross over operation without the process of adding or removing potential solution from the league, this can be referred as HMLCA-V1. In an another embodiment, the system 100 can perform uniform cross over operation in tandem with adding and removing potential solution, this version of present disclosure can be referred as HMLCA-V2. In yet another embodiment, the system 100 can use another set of cross over operation with or without addition and removal of potential solutions. In the present disclosure, the original work of league championship algorithm (which is conventionally known) is referred as LCA which can be obtained at 'Kashan, Ali Husseinzadeh. "League championship algorithm: a new algorithm for numerical function optimization." 2009 International Conference of Soft Computing and Pattern Recognition. IEEE, 2009'.

The below table presents various terms used in the present disclosure and their relation to the original work LCA referenced above. These terms are used interchangeably throughout the disclosure.

*Table 4: Glossary of terms*

| Literature terminologies (League Championship Algorithm) as known in the conventional art | Meaning as known in the conventional art | Term used hereinafter in the present disclosure |
|---|---|---|
| Team formation | Solution (a set of values where each value corresponds to a parameter of the specified mathematical model) of a mathematical model | Potential Solution (solution constrained by parameter boundary) |
| League | Population of teams/solutions | Population of potential solutions |
| League size | No of teams/solutions in the league | Population size (number of potential solutions) |
| Team I | $i^{th}$ team/solution of the population/league | $i^{th}$ potential solution of the population |
| Number of seasons | First level of iterations | Number of seasons |
| Number of week | Second level of iterations. It is determined in terms of no of teams/solutions in the league | Number of week |
| League schedule | Pairing of teams/solutions so that each team/solution will play against all other teams/solutions exactly once throughout the seasons and will play only one game in a week. | Pairing of potential solutions |
| Team's current best formation | So far found best solution for the corresponding solution/team | Potential solution's current optimal parameter values |
| Winner and loser | When two teams play against each other, one team will win the match and is called winner and the other team is called loser | Type I potential solution (winner) and Type II potential solution (loser) |
| Playing strength | Function or fitness value | Fitness value or Objective function |
| Teams' best formation | So far found best solution | global optimal solution |

(continued)

| Literature terminologies (League Championship Algorithm) as known in the conventional art | Meaning as known in the conventional art | Term used hereinafter in the present disclosure |
|---|---|---|
| Optimal function value | So far found best fitness value across teams/solutions | Fitness value of global optimal solution |

In an embodiment of the present disclosure, the below Table 5 defines various control parameters of HMLCA that can be altered to obtain desired results from the process. The values of these parameters are also critical to the effectiveness or success of the algorithm. League championship algorithm has following control parameters: league size/population size, number of seasons, probability of success ($p_c$) and rate of increase of $p_c$. League size determines the number of solutions to be generated to form the population. In an example embodiment number of seasons can be used as a stopping criteria. Probability of success ($p_c$) is used to determine the number of parameters to be updated in a solution. The larger the value of $p_c$ the lesser the number of parameter updations in a potential solution. Rate of increase of $p_c$ is used to increase the value of $p_c$ after each week which in turn decreases the number of updation.

*Table 5: Control parameters of present disclosure*

| Name | Definition | Example |
|---|---|---|
| League size (L) | Number of initial solutions or population size | 40 |
| No of seasons (S) | One of the stopping criteria: number of iterations | 100 |
| Probability of success ($p_c$) | Parameter that determines the number of parameters to be updated in a solution | 0.8 |
| Rate of increase of $P_c$ ($\alpha$) | Parameter that increases the value of $p_c$ after each week which in turn decreases the number of updation in a solution. | 1.005 |

The present disclosure is further explained in detail using a case study, sourced from 'Gabrielsson, Johan, and Daniel Weiner. Pharmacokinetic and pharmacodynamic data analysis: concepts and applications. 4th Edition. CRC Press, 2006", that uses pharmacokinetic data from several species to estimate allometric parameters. The results from the parameter estimation or optimization methods are used to assess superimposibility, model cl, $V_c$, $V_t$, dose, AUC and other related values or inferences of interest. The study assesses the pharmacokinetics of a drug in five species, whose details are given in the table 6 below.

*Table 6: Case Study Dose and Species Details*

| Species | Mouse | Rat | Monkey | Dog | Man |
|---|---|---|---|---|---|
| Body Weight (BW) | 20 g | 250 g | 3.5 kg | 14 kg | 70 kg |
| Dose (in $\mu$g) | 10 | 125 | 200 | 6000 | 12000 |

In the present exemplary case study, plasma concentration time profile of the drug in all species, given in table 6, was collected at regular intervals and is given in Table 7 below. This data was one of the inputs to the present disclosure.

*Table 7: PCT profile of all species used in case study*

| Time (hr) | Concentration (Conc.) ($\mu$g/L) in Man | Conc. ($\mu$g/L) in Dog | Conc. ($\mu$g/L) in Monkey | Conc. ($\mu$g/L) in Rat | Conc. ($\mu$g/L) in Mouse |
|---|---|---|---|---|---|
| 2 | 600 | 1300 | 200 | 900 | 950 |
| 5 | 450 | 800 | 40 | 400 | 400 |
| 10 | 170 | 250 | 48 | 460 | 300 |
| 20 | 98 | 260 | 27 | 200 | 30 |
| 40 | 55 | 150 | 29 | 130 | 4.5 |

(continued)

| Time (hr) | Concentration (Conc.) (μg/L) in Man | Conc. (μg/L) in Dog | Conc. (μg/L) in Monkey | Conc. (μg/L) in Rat | Conc. (μg/L) in Mouse |
|---|---|---|---|---|---|
| 60 | 70 | 187 | 17 | 25 | 0.1 |
| 90 | 45 | 130 | 17 | 15 | - |
| 120 | 52 | 120 | 6 | 1 | - |
| 480 | 10 | 40 | 2.5 | - | - |
| 600 | 10 | 5 | - | - | - |
| 1440 | 0.38 | - | - | - | - |

The plasma concentration data of Table 7 was modelled using the below equations and the seven parameters of the model are estimated by minimizing or maximizing one or more of the fitness or objective function values. The parameters to be estimated are: a, b, c, d, e, and g. The model equations are:

$$cl = a * BW^b$$

$$cl_d = g * BW^b$$

$$V_c = c * BW^d$$

$$V_t = e * BW^d$$

$$K_{10} = \frac{cl}{V_c}$$

$$K_{12} = \frac{cl_d}{V_c}$$

$$K_{21} = \frac{cl_d}{V_t}$$

$$K = K_{10} + K_{12} + K_{21}$$

$$R = (K^2 - 4 * K_{21} * K_{10})^{0.5}$$

$$alpha = \frac{K + R}{2}$$

$$beta = \frac{K - R}{2}$$

$$coeff = \frac{Dose}{V_c}$$

$$C(t) = \frac{Dose}{V_c} * \frac{(alpha - K_{21})}{alpha - beta} * e^{-alpha*t} - \frac{Dose}{V_c} * \frac{(beta - K_{21})}{(alpha - beta)} * e^{-beta*t} \quad ---$$

equation (6)

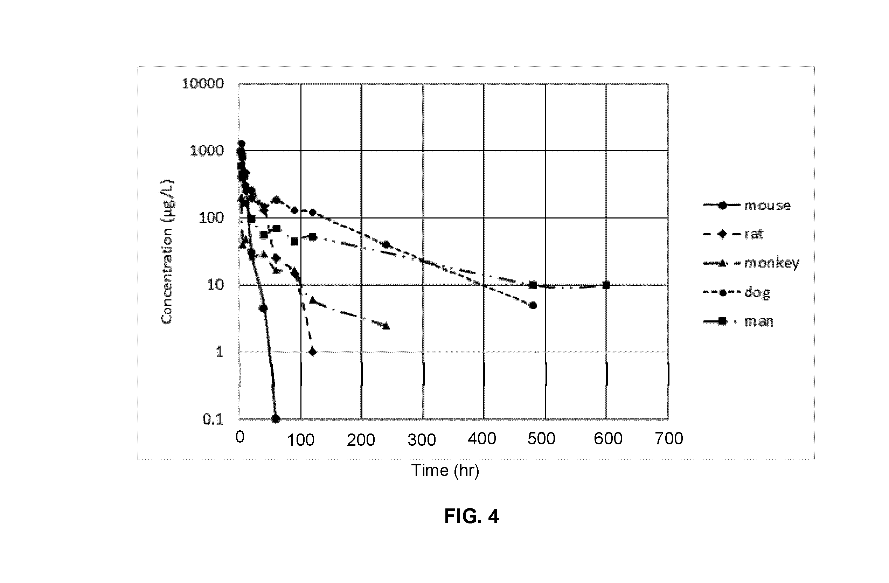

where C(t) is the concentration at time point t for the corresponding species. More particularly, FIG. 4, with reference to FIGS. 2 through 3B, depicts a graphical representation of the (PCT) profile of the administered doses in accordance with an example embodiment of the present disclosure. For the sake of brevity, the graphical representation does not depict 1440 time (hr) and 0.38 concentration values. Additionally, the parameter boundaries used for this case study can be as follows:

*Table 8: Case Study Parameter Boundaries*

| Parameter | Lower Bound | Upper bound |
|-----------|-------------|-------------|
| a | 0 | 0.5 |
| b | 0 | 2 |
| c | 0 | 25 |
| d | 0 | 2 |
| e | 0 | 25 |
| g | 0 | 0.08 |

In the example embodiment, the present disclosure minimizes weighted residual sum of squares value (WRSS), fitness value or objective function to estimate the seven pharmacokinetic parameters. Fitness function used: Fitness/ Objective Function /

$$WRSS = \sum_{j=1}^{d} \sum_{i=1}^{n_j} W_{ij}(Obs_{ij} - Pred_{ij})^2 \quad \text{equation (7)}$$

where d is the number of data sets, $n_j$ is the number of observations for $j^{th}$ data set, $Obs_{ij}$ is the $i^{th}$ observed value for $j^{th}$ data set, $Pred_{ij}$ is the predicted value of $i^{th}$ observation for $j^{th}$ data set, and

$$W_{ij} = \frac{1}{Obs_{ij}},$$

for weighted least square

$$W_{ij} = \frac{1}{Pred_{ij}},$$

for iteratively reweighted least square

In the current example embodiment, the set of parameters used by present disclosure, namely, league size, no of seasons, probability of success ($p_c$), a rate of increase of $p_c$ can be set as given in Table 9 below.

Table 9: *Case Study HMLCA Parameters*

| Control Parameters | Values |
|--------------------|--------|
| League size (L) - Number of initial solutions or population size | 40 |
| No of seasons (S) | 10 |
| Probability of success ($p_c$) | 0.8 |
| Rate of increase of $p_c$ ($\alpha$) | 1.005 |

The present disclosure estimates the parameters a, b, c, d, e, and g using a) the PCT data, given in Table 7, b) dosing and species data, given in Table 6, c) model equations given in equation (6), d) parameter boundaries given in table 8, and e) a selected fitness or objective function, as per equation (7). The information (a) to (e) is received by the system 100 through the one or more hardware processors 104. Further, the system 100, HMCLA or the present disclosure is configured as per the control parameters given in Table 9, to obtain the optimal parameters for the current case study.

In the current example embodiment, the system 100 randomly generates initial set of potential solutions, referred as population, by using the present disclosure, the input, and the control parameters given above as per the equation (2). A sample of those generated population at step 204 is presented in the below Table 10 by way of example.

*Table 10: Sample initial set of potential solutions (or population)*

| Solution(s) | Parameter value (team formation) [a, b, c, d, e, g] |
|---|---|
| Solution 1 | [0.3952, 1.9306, 6.0122, 1.5665, 2.0668, 0.0121] |
| Solution 2 | [0.0752, 1.3544, 11.7360, 1.3273, 16.2335, 0.0359] |
| Solution 3 | [0.0953, 0.9771, 13.7090, 1.0581, 1.4539, 0.0125] |
| Solution 38 | [0.0944, 0.0192, 23.6517, 1.1932, 0.8952, 0.0367] |
| Solution 39 | [0.4652, 0.5297, 3.3199, 1.4978, 14.4920, 0.0301] |
| Solution 40 | [0.4681, 0.6466, 1.9966, 0.9440, 4.1595, 0.0061] |

In the above example, feasible/potential solution, consisting of the value of each parameter within the specified boundary, can be referred as 'team formation'. System 100 generates 40 potential solutions as the league size control parameter to the HMLCA is 40.

In the present example embodiment, the fitness of each potential solution has been evaluated using WRSS, calculated as per equation (7). Lower the WRSS value, fitter the potential solution. Hence, the objective of HMLCA of the present disclosure is to find an optimal potential solution with least value of WRSS. Following table (Table 11) depicts a fitness value being computed for each of the potential solutions discussed above, as an example. Subsequently system 100, in the first iteration, assigns each of the randomly generated solutions depicted in Table 10 as the current optimal formation for each solution of the population.

*Table 11: Sample WRSS and Current Optimal Formation of potential solutions*

| Solution(s) | WRSS (fitness value) | Current optimal team formation/ parameter value |
|---|---|---|
| Solution 1 | 1.8927E18 | [0.3952, 1.9306, 6.0122, 1.5665, 2.0668, 0.0121] |
| Solution 2 | 394940.33 | [0.0752, 1.3544, 11.7360, 1.3273, 16.2335, 0.0359] |
| Solution 3 | 221045.50 | [0.0953, 0.9771, 13.7090, 1.0581, 1.4539, 0.0125] |
| Solution 38 | 4165116.43 | [0.0944, 0.0192, 23.6517, 1.1932, 0.8952, 0.0367] |
| Solution 39 | 2303728.07 | [0.4652, 0.5297, 3.3199, 1.4978, 14.4920, 0.0301] |
| Solution 40 | 3977985.05 | [0.4681, 0.6466, 1.9966, 0.9440, 4.1595, 0.0061] |

In the present example, global best solution is determined based on the WRSS of the potential solutions in the population (which comprises of 40 solutions). After initializing the current optimal potential solutions for population, system 100, learns the global optimal solution among the league as [0.0909, 0.9929, 2.4210, 0.7539, 11.9116, 0.0083] with fitness value i.e., WRSS as 129252.1153.

In the current example embodiment, the number of seasons (S) is used as a stopping criteria i.e., the stopping criteria is used here to define the number of times steps 210 to 224 would be carried out. In the present case study, the value of S is 10.

In the case study as described herein, at step 210 of the present disclosure, the one or more hardware processors 104 pair the potential solutions where all the potential solutions are paired against all other potential solutions exactly once. Pairings of solutions are decided wherein a comparison with each other is performed at different time intervals of next updation process. Following table (Table 12) depicts paring of solutions for a first pre-determined interval (e.g., say week 1):

*Table 12: Case study sample pairing of solutions*

| **Pair 1** | **Pair 2** | **Pair 3** | **Pair 4** | **Pair 5** |
|---|---|---|---|---|
| Solution 1 | Solution 2 | Solution 3 | Solution 4 | Solution 5 |

(continued)

| Pair 1 | Pair 2 | Pair 3 | Pair 4 | Pair 5 |
|---|---|---|---|---|
| Solution 40 | Solution 39 | Solution 38 | Solution 37 | Solution 36 |

At step 212 of the present disclosure, the one or more hardware processors 104 determine a type I potential solution and type II potential solution from each of the unique pairs for the current iteration, using a fitness value associated with (i) each potential solution of the pairs and (ii) the global optimal solution. In an embodiment, type I potential solution is a winner, and type II potential solution is a loser. The step 212 is preceded by a step of whether maximum time interval is reached. As the number of potential solutions is 40 the maximum time interval in the current case study is 39 weeks. Below table (Table 13) depicts fitness value (WRSS based) for particularly solutions pair (say Solution 7 and Solution 34):

*Table 13: Sample pairing of solutions in case study with fitness values*

| Pair 7 | WRSS |
|---|---|
| Solution 7 | 1892652.2297 |
| Solution 34 | 931574.8203 |

In the considered example, with solution 7 and solution 34, to simulate win or lose, a random number is generated between 0 and 1. If it is less than or equal to $P_I^T$, then solution 7 wins the contest. Otherwise solution 34 wins. The value of $P_I^T$ is calculated using equation 5 discussed above. For the current case study being discussed in the present disclosure, the value of $P_I^T$ is (using solution 7 and 34) 0.312708. It is noted from the above and experimental results that due to associated randomness in the calculation, Solution 7 was picked up as better solution in spite of having higher WRSS value.

In the considered example of the present disclosure, solution 7 was picked up as winner even though solution 34 has better fitness value. Therefore, at step 214 of the present disclosure, the one or more hardware processors 104 perform a uniform cross over to minimize the drawbacks of obtaining solution 7 as winner, as discussed prior/earlier in the present disclosure. The result of crossover is given below table (Table 14) provided by way of example:

*Table 14: Result of Uniform Crossover on Solution 7 and 34*

| Paired solutions | Parameter Value | Parameter value after crossover |
|---|---|---|
| **Solution 7** | [0.4576, 0.8749, 22.6326, 1.6031, 19.0645, 0.0039] | [0.4576, 0.8749, 7.6651, 1.6031, 22.8366, 0.0177] |
| **Solution 34** | [0.2924, 0.6407, 7.6651, 1.5533, 22.8366, 0.0177] | [0.2924, 0.6407, 22.6326, 1.5533, 19.0645, 0.0039] |

At step 216 of the present disclosure, the one or more hardware processors 104 generate new potential solutions based on the optimized fitness value of the type I potential solution and type II potential solution. Below is an exemplary table (Table 15) depicting new solutions after the first pre-determined time interval (e.g., say week 1):

*Table 15: Result of Updating (updated) Population in Week 1 at step 216*

| Solution | Parameter value (new solution formation) | WRSS |
|---|---|---|
| Solution 1 | [0.3952, 1.9306, 6.0122, 1.4917, 2.0668, 0.0121] | 3.7155E24 |
| Solution 2 | [0.0752, 1.3544, 11.7360, 1.3273, 16.2335, 0.0354] | 394843.86 |
| Solution 3 | [0.0953, 0.9771, 13.7090, 1.0581, 1.4539, 0.0037] | 220433.07 |
| Solution 38 | [0.0944, 0.0192, 23.6517, 1.1932, 1.2088, 0.03678] | 3325733.96 |
| Solution 39 | [0.4652, 1.1280, 3.3199, 1.4978, 14.4920, 0.0301] | 845929.65 |
| Solution 40 | [0.4681, 0.6466, 1.9966, 0.5496, 4.1595, 0.0061] | 3125563.47 |

At step 218 of the present disclosure, the one or more hardware processors 104 perform a comparison of a fitness value of the parameter values corresponding to the new solutions with a fitness value of (i) current optimal parameter values of the new solutions and (ii) the global optimal solution, and based on this comparison the current optimal parameter values and the global optimal solution (e.g., global best solution) are updated with the plurality of the new parameter values. Below example depicts global optimal solution after 1st week:
[0.0231, 1.5688, 3.3656, 1.1347, 22.9350, 0.0486] with WRSS = 99931.1499.

In the example case study of the present disclosure, at step 220, the one or more hardware processors 104 eliminate a fixed (10% of initial solutions generated) number of sub-optimal solutions or worst performing solutions. After each week 10 % of the total solution i.e., 4 solutions were removed from the population. These 4 solutions had the greatest WRSS value and did not fit the observed PCT data optimally to the model. The removed worst (also referred as 'sub-optimal' and interchangeably used hereinafter) 4 solutions are given in table below (Table 16) by way of example:

Table 16: Sub-optimal solutions removed from the population

| Solution | Parameter value | WRSS |
|---|---|---|
| Solution 3 | [0.3764, 1.4606, 13.6153, 0.0035, 14.3209, 0.0342] | 6.1051E295 |
| Solution 9 | [0.1891, 1.1156, 15.3163, 0.2170, 4.6780, 0.0337] | 9.7921E158 |
| Solution 30 | [0.1859, 0.1917, 24.8027, 1.9949, 4.1572, 0.0136] | 2.8183E84 |
| Solution 29 | [0.3251, 1.5791, 15.9194, 0.7962, 9.4126, 0.0478] | 2.0602E76 |

In yet an embodiment of the case study, at step 222, the one or more hardware processors 104 add four randomly generated solutions to the population. To maintain the fixed size and diversity of the league, same number, as the ones removed, of randomly generated solutions (teams) are added to the league. The new solutions that were added are given in table below (Table 17) by way of example:

Table 17: Sample new solutions added to the population

| New Solution | Parameter value | WRSS |
|---|---|---|
| Solution 3 | [0.1090, 0.6575, 11.0022, 1.9221, 21.6251, 0.0369] | 2947107.6423 |
| Solution 9 | [0.1449, 0.0436, 16.4565, 0.5911, 24.2936, 0.0273] | 369731.844 |
| Solution 30 | [0.0632, 0.6008, 22.3493, 1.1185, 12.5054, 0.0485] | 411982.4032 |
| Solution 29 | [0.1411, 1.6648, 18.0303, 1.1491, 11.3806, 0.0413] | 7.25E+013 |

It can be inferred from Table 16 and 17 that the process of deleting and adding potential solutions to the league improves the quality of solutions and helps in faster convergence of the optimization process.

At step 224 of the present disclosure, the one or more hardware processors 104 generate a global optimal solution from the updated population after the completion of the predefined number of iterations. In the present disclosure, parameters with minimum WRSS value form an optimal solution that is generated from the updated population. Below is an example of the optimal potential solution (e.g., best potential solution) with parameters having minimum WRSS:
IP_GS = [0.00884, 0.5849, 0.5890, 0.8709, 16.8520, 0.0100], WRSS = 3585.6903

At step 226 of the present disclosure, the one or more hardware processors 104 perform a local optimization technique called as Nelder Mead on the global optimal solution, IP_GS, as initial value based on which parameter value with minimum WRSS value is achieved. Below is example of set of optimized parameter values (PK parameter values) for PK model which is being obtained in step 202 and described as a use case scenario by the present disclosure:
**[0.0208, 0.7556, 0.1038, 1.19642, 0.5436, 0.0679], WRSS = 304.8978.**

Below tables (Table 18 and 19) depicts result comparison of literature version of LCA and Hybrid Modified LCA (present disclosure):

*Table 18: Exemplary Case Studies Model Details*

| S. No. | Model and Dosing Information | Number of Parameters | Model Equation |
|---|---|---|---|
| 1 | One compartmental model with single intravenous bolus dose ($D_{iv}$) | 2 (V, $K_{10}$) | $$C = \frac{D_{iv}}{V} \times e^{-K_{10}.t}$$ |
| 2 | Turnover model with no lag-time to model<br><br>Activity of prothrombin complex activity (PCA) after intravenous bolus dosing. | 4 ($IC_{50}$, $k_{out}$, n, P0) | $$\frac{dPCA}{dt} = \frac{dR}{dt} = k_{out}(P0 * INHIB - R)$$ $$INHIB = \frac{1}{1 + (CW/IC_{50})^n}$$ Where, $$CW = constant_1 \times e^{-constant_2 \times t}$$ |
| 3 | Two compartmental model with constant intravenous infusion dosing | 5 ($V_c$, $Cl_d$, $V_t$, $V_{max}$, $K_m$) | Rate of change of concentration of drug in central compartment: $$\frac{dC}{dt} = \frac{In - Cl.C - Cl_d.C + Cl_d.C_t}{V_c}$$ Rate of change of concentration of drug in peripheral compartment: $$\frac{dC_t}{dt} = \frac{Cl_d.C - Cl_d.C_t}{V_t}$$ Here, $$Here, Cl = \frac{V_{max}.C}{K_m + C}$$ $$In = \frac{D_{inf}}{T_{inf}} \ for \ T \le T_{inf}$$ and $In = 0$ for $T > T_{inf}$ |
| 4 | One compartmental model with zero-order input and transdermal input | 5 (V, Cl, $Dose_{inf}$, $T_{fst}$, $T_{inf}$) | $$\frac{dC}{dt} = \frac{F_{inf} + F_{fst} - Cl.C}{V}$$ Here, $$F_{inf} = \frac{Dose_{inf}}{Time \ Period_{inf}} \ for \ T \le T_{inf},$$ and $F_{inf} = 0$ for $T > T_{inf}$ $$F_{fst} = \frac{Dose_{fst}}{T_{fst}} = \frac{Released \ dose - Dose_{inf}}{Time \ period_{fst}},$$ for $T \le T_{fst}$, and $F_{fst} = 0$ for $T > T_{inf}$ |
| 5 | Three compartmental model with single intravenous bolus dosing | 6 (A, B, C, $\alpha$, $\beta$, $\gamma$) | Concentration of drug in plasma: $$C_p = Ae^{-\alpha.t} + Be^{-\beta.t} + C \, e^{-\gamma.t}$$ |

(continued)

| S. No. | Model and Dosing Information | Number of Parameters | Model Equation |
|---|---|---|---|
| 6 | Simultaneously fitting data from mouse, rat, monkey, dog and man with intravenous bolus dosing using multi-compartment allometric scaling | 6 (a, b, c, d, e, g) | $C_t = \frac{Dose}{V_c} \times \frac{\alpha - K_{21}}{\alpha - \beta} \times e^{-\alpha.t} - \frac{Dose}{V_c} \times \frac{\beta - K_{21}}{\alpha - \beta} \times e^{-\beta.t}$ <br><br> Where, <br> Dose = intravenous bolus dose for one of the species <br> BW = body weight of the concerned species <br> $cl = a.BW^b$ <br> $cl_d = g.BW^b$ <br> $V_c = c.BW^d$ <br> $V_t = e.BW^d$ <br> $K_{10} = \frac{cl}{V_c}, \; K_{12} = \frac{cl_d}{V_c}, \; K_{21} = \frac{cl_d}{V_t}$ <br> $\alpha = \frac{K_{10} + K_{12} + K_{21} + \sqrt{K^2 - 4.K_{21}.K_{10}}}{2}$ <br> $\beta = \frac{K_{10} + K_{12} + K_{21} - \sqrt{K^2 - 4.K_{21}.K_{10}}}{2}$ |
| 7 | Differential equation model for reversible metabolism | 6 ($V_p$, $Cl_m$, $V_m$, $Cl_p$, $K_{12}$, $K_{21}$) | Rate of change of concentration of parent when parent compound injected: <br> $\frac{dC_p}{dt} = \frac{Input_p}{V_p} - K_{12}C_p - \frac{Cl_p}{V_p}.C_p + K_{21}.C_m$ <br> Rate of change of concentration of metabolite compound when metabolite was injected or metabolite came along with parent injection: <br> $\frac{dC_m}{dt} = \frac{Input_m}{V_m} - K_{12}C_m - \frac{Cl_m}{V_m}.C_m + K_{21}.C_p$ <br> Rate of change of concentration of parent compound when metabolite was injected <br> $\frac{dC_p}{dt} = - K_{12}C_p - \frac{Cl_p}{V_p}.C_p + K_{21}.C_m$ <br> Where, <br> $Input = \frac{Dose}{T_{inf}} \; for \; 0 < T \leq T_{inf},$ <br> and <br> Input = 0 for $T > T_{inf}$ |

(continued)

| S. No. | Model and Dosing Information | Number of Parameters | Model Equation |
|---|---|---|---|
| 8 | Enterohepatic model with intravenous bolus dosing | 7 ($V_c$, $Cl$, $Cl_d$, $V_t$, $K_a$, $K_{1g}$, $T_{tom}$) | Rate of change of drug in central compartment:<br><br>$$\frac{dC}{dt} = \frac{K_a A_g - Cl.C - Cl_d.C + Cl_d.C_t - K_{1g}.}{V_c}$$<br><br>Rate of change of drug in peripheral compartment:<br><br>$$\frac{dC_t}{dt} = \frac{Cl_d.C - Cl_d.C_t}{V_t}$$<br><br>If $RI < T \le (RI + T_{tom})$ then<br>Rate of change of drug in Gut:<br><br>$$\frac{dC_g}{dt} = \frac{A_{bile}}{\tau} - K_a A_g$$<br><br>Rate of change of drug in bile:<br><br>$$\frac{dC_b}{dt} = K_{1g}.C.V_c - \frac{A_{bile}}{\tau}$$<br><br>In all other scenarios,<br><br>$$\frac{dC_g}{dt} = - K_a A_g$$<br><br>$$\frac{dC_b}{dt} = K_{1g}.C.V_c$$ |

*Table 19: Results of present disclosure and prior art (or conventional LCA) for Exemplary Case Studies*

| S. No. | No. of Parameters | Best fitness value (WRSS) achieved | |
|---|---|---|---|
| | | **LCA-V** | **HMLCA-V** |
| 1 | 2 | 4327.38 | 4327.38 |
| 2 | 4 | 3586.76 | 152.94 |
| 3 | 5 | $71.6 \times 10^{-3}$ | $19.3 \times 10^{-3}$ |
| 4 | 5 | 0.44 | 0.24 |
| 5 | 6 | $3.68 \times 10^{-3}$ | $3.68 \times 10^{-3}$ |
| 6 | 6 | 865.01 | 304.87 |
| 7 | 6 | 77.35 | 0.089 |
| 8 | 7 | 572.44 | 0.146 |

In the above table, LCA refers to Original version of LCA proposed and conventionally known in the literature, LCA-V refers to LCA followed by Nelder Mead/Gauss Newton local optimization technique, HMLCA-V refers to LCA with Step 214 (Perform crossover), Step 220 (Remove worst teams) and Step 222 (Add new teams) followed by Nelder Mead or Gauss Newton optimization technique. The above table demonstrates the technical solution presented by the present disclosure in solving one or more technical problems discussed in herein. It can be observed that the prior art, LCA, with same inputs and parameters fail to yield optimal Pharamacokinetic or Pharamacodynamic parameters. This may be attributed to the problem of local optimization, poor quality teams, reduced diversity in the league, slower convergence rates, etc.

In an embodiment, the present disclosure can be applied to varied set of models and application. Some of the classes of PK studies are a) IV bolus, oral and constant infusion dosing, b) single, multiple and simultaneous dosing, c) simultaneous plasma and urine analysis, d) algebraic and differential equation based models, e) one, two and three compartment models and f) number of parameters varies from 2 to 11. Below are the applications (Table 20) of these PK/PD case studies:

*Table 20: Sample Practical Applications of Present Disclosure*

| Pharmacokinetic Applications |
| --- |
| One-compartment iv bolus dosing |
| One-compartment oral dosing |
| One-compartment $1^{st}$ and 0-order input |
| One-compartment iv plasma/urine |
| Two-compartment iv bolus dosing |
| Two-compartment distribution models |
| Two-compartment model testing |
| Simultaneous fitting of iv/oral data |
| Two-compartment repeated oral dosing |
| Bolus plus constant rate infusion |
| Multi-compartment model oral dosing |
| Toxicokinetics |
| Nonlinear kinetics - Capacity I |
| Ethanol kinetics - Capacity IV |
| Nonlinear kinetice - Capacity IV |
| Nonlinear kinetics - Heteroinduction |
| Two-compartment plasma and urine analysis with rate and ARE plots |
| Allometry - Complex Dedrick plot |
| Turnover I - Sc dosing of hormone |
| Turnover II - IV dosing of hormone |
| Transdermal input and kinetics |
| Reversible metabolism |
| Bayesian model - Digoxin |
| Time controlled drug delivery |
| Two-compartment plasma data - Experimental design issues |
| Enterohepatic recirculation |
| Multiple intravenous infusions - NCA versus regression |
| Saturable absorptions via transporters |
| Multi-compartment drug/metabolite |
| Impact of disease on r-hSOD kinetics |
| **Pharmacodynamics Applications** |
| Turnover model 1 - Bolus dosing |
| Turnover model 2 - IV infusions |
| Turnover model 3 - Turnover versus link modeling |
| Turnover model 4 - IV infusions |

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure with a true scope of disclosed embodiments being indicated by the following claims.

## Claims

1. A processor implemented method, comprising:

   obtaining, via one or more hardware processors, using a Hybrid Modified League Championship Algorithm, HMLCA, a set of data pertaining to one of a Pharmacokinetics, PK, model or a Pharmacodynamics, PD, model, dosage information associated with the set thereof, a concentration-time or a response-time data, and a parameter boundary for the PK model or the PD model, wherein the set of data comprises parameters values corresponding to one of PK parameters or PD parameters (202);
   randomly generating, using the HMLCA, a set of potential solutions as a population, based on the parameter boundary and assigning generated parameter values as a current best formation for each potential solution from the set, wherein each potential solution from the population comprises parameter values that are within the parameter boundary (204);
   computing, using the HMLCA, a fitness value for each potential solution of the population based on one or more criteria (206);
   computing, using the HMLCA, a global optimal solution among the population, wherein the global optimal solution comprises optimal parameter values from initialized parameter values (208);
   pairing, using the HMLCA, until a stopping criteria is satisfied, the potential solutions across each other based on a predefined rule to obtain unique pairs of potential solutions, wherein the unique pairs of potential solutions are being identified for (i) comparison against each other and (ii) subsequent updation of each potential solution (210), and wherein the stopping criteria is defined that is indicative of number of times each solution needs an update;
   determining, using the HMLCA, using a fitness value associated with (i) each potential solution from the unique pairs of potential solutions and (ii) the global optimal solution, a type I potential solution and a type II potential solution from each of the unique pairs (212);
   optimizing, using the HMLCA, the fitness value of the type I potential solution and type II potential solution by performing a crossover of one or more parameter values associated thereof (214);

generating, using the HMLCA, new potential solutions based on a current optimal parameter value of the type I potential solution and type II potential solution (216);

performing, using the HMLCA, a comparison of a fitness value of the parameter values corresponding to the new solutions with a fitness value of (i) current optimal parameter values of the new solutions and (ii) the global optimal solution and updating, based on the comparison, the current optimal parameter values with the parameter values for each new solution and the global optimal solution (218);

eliminating, using the HMLCA, a subset of the new solutions based on a comparison of a fitness value of each of the new solutions with a fitness threshold to obtain a filtered set of solutions (220);

adding, using the HMLCA, a new set of randomly generated potential solutions into the filtered set of potential solutions to obtain an updated population (222);

generating, using the HMLCA, a global optimal solution from the updated population based on an optimal fitness value (224); and

performing, using the HMLCA, a local optimization technique on the global optimal solution to estimate a set of optimized parameter values (226).

2. The processor implemented method of claim 1, wherein each solution comprised in the filtered set of solutions includes optimized parameter values, and wherein each of the optimized parameter values comprises a fitness value that is less than or equal to the fitness threshold.

3. A system (100) comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

obtain, using a Hybrid Modified League Championship Algorithm, HMLCA, a set of data pertaining to one of a Pharmacokinetics, PK, model or a Pharmacodynamics, PD, model, dosage information associated with the set thereof, a concentration-time or a response-time data, and a parameter boundary for the PK model or the PD model, wherein the set of data comprises parameter values corresponding to one of PK parameters or PD parameters;

randomly generate, using the HMLCA, a set of potential solutions as a population based on the parameter boundary and assign generated parameter values as current best formation for each potential solution from the set, wherein each solution from the set of potential solutions comprises parameter values that are within the parameter boundary;

compute, using the HMLCA, a fitness value for each potential solution of the population based on one or more criteria;

compute, using the HMLCA, a global optimal solution among the population, wherein the global optimal solution comprises optimal parameter values from initialized parameter values;

pair, using the HMLCA, until a stopping criteria is satisfied, the potential solutions across each other based on a predefined rule to obtain unique pairs of potential solutions, wherein the to obtain unique pairs of potential solutions are being identified for (i) comparison against each other and (ii) subsequent updation of each potential solution, and wherein a stopping criteria is defined that is indicative of number of times each solution needs an update;

determine, using the HMLCA, using a fitness value associated with (i) each potential solution from the unique pairs of potential solutions and (ii) the global optimal solution, a type I potential solution and a type II potential solution from each of the unique pairs;

optimize, using the HMLCA, the fitness value of the type I potential solution and type II potential solution by performing an uniform crossover of one or more parameter values associated thereof;

generate, using the HMLCA, new potential solutions based on a current optimal parameter value of the type I potential solution and type II potential solution;

perform, using the HMLCA, a comparison of a fitness value of the parameter values corresponding to the new solutions with a fitness value of (i) current optimal parameter values of the new solutions and (ii) the global optimal solution and update, based on the comparison, the current optimal parameter values with the parameter values for each new solution and the global optimal solution;

eliminate, using the HMLCA, a subset of the new solutions based on a comparison of a fitness value of each of the new solutions with a fitness threshold to obtain a filtered set of solutions;

add, using the HMLCA, a new set of randomly generated potential solutions into the to obtain a filtered set of solutions to obtain an update population;

generate, using the HMLCA, a global optimal potential solution from the updated population; and

perform, using the HMLCA, a local optimization technique on the optimal potential solution to estimate a set of optimized parameter values.

4. The system of claim 3, wherein each solution comprised in the filtered set of solutions includes optimized parameter values, and wherein each of the optimized parameter values comprises a fitness value that is less than or equal to the fitness threshold.

5. One or more non-transitory machine readable information storage mediums comprising one or more instructions which when executed by one or more hardware processors causes generation of optimized set of Pharmacokinetic, PK, and Pharmacodynamic, PD, parameters by:

obtaining, using a Hybrid Modified League Championship Algorithm, HMLCA, a set of data pertaining to one of a Pharmacokinetics, PK, model or a Pharmacodynamics, PD, model, dosage information associated with the set thereof, a concentration-time or a response-time data, and a parameter boundary for the PK model or the PD model, wherein the set of data comprises parameters values corresponding to one of PK parameters or PD parameters;

randomly generating, using the HMLCA, a set of potential solutions as a population based on the parameter boundary and assigning generated parameter values as a current best formation for each potential solution from the set, wherein each of the potential solutions from the set comprises parameter values that are within the parameter boundary;

computing, using the HMLCA, a fitness value for each of the potential solutions based on one or more criteria, wherein the fitness value is calculated in the form of an objective function, and wherein the one or more criteria comprises at least one of strength and weakness pertaining to each of the plurality of potential solutions;

computing, using the HMLCA, a global optimal solution among the potential solutions/population, wherein the global optimal solution comprises optimal parameter values from initialized parameter values;

pairing, using the HMLCA, until a stopping criteria is satisfied, the potential solutions across each other based on a predefined rule to obtain unique pairs of potential solutions, wherein the unique pairs of potential solutions are being identified for (i) comparison against each other and (ii) subsequent updation of each potential solution, and wherein the stopping criteria is defined that is indicative of number of times each solution needs an update;

determining, using the HMLCA, using a fitness value associated with (i) each potential solution from the unique pairs of potential solutions and (ii) the global optimal solution, a type I potential solution and type II potential solution from each of the unique pairs;

optimizing, using the HMLCA, the fitness value of the type I potential solution and type II potential solution by performing a crossover of one or more parameter values associated thereof;

generating, using the HMLCA, new potential solutions based on the current optimal parameter value of the type I potential solution and type II potential solution;

performing, using the HMLCA, a comparison of a fitness value of the parameter values corresponding to the new solutions with a fitness value of (i) current optimal parameter values of the new solutions and (ii) the global optimal solution and updating, based on the comparison, the current optimal parameter values with the parameter values for each new solution and the global optimal solution;

eliminating, using the HMLCA, a subset of the new solutions based on a comparison of a fitness value of each of the new solutions with a fitness threshold to obtain a filtered set of solutions, wherein each solution comprised in the filtered set of solutions includes optimized parameter values, and wherein each of the optimized parameter values comprises a fitness value that is less than or equal to the fitness threshold;

adding, using the HMLCA, a new set of randomly generated potential solutions into the filtered set of solutions to obtain an updated population;

generating, using the HMLCA, a global optimal solution from the updated population based on an optimal fitness value; and

performing, using the HMLCA, a local optimization technique on the global optimal solution to estimate a set of optimized parameter values.

6. The one or more non-transitory machine readable information storage mediums of claim 5, wherein each solution comprised in the filtered set of solutions includes optimized parameter values.

7. The one or more non-transitory machine readable information storage mediums of claim 6, wherein each of the optimized parameter values comprises a fitness value that is less than or equal to the fitness threshold.

**Patentansprüche**

1. Prozessorimplementiertes Verfahren, umfassend:

Erhalten, über einen oder mehrere Hardwareprozessoren, unter Verwendung eines Hybrid Modified League Championship Algorithmus, HMLCA, eines Satzes von Daten, die sich auf eines von einem Pharmakokinetik-, PK-, Modell oder einem Pharmakodynamik-, PD-, Modell, Dosierungsinformationen, die dem Satz davon zugeordnet sind, Konzentrationszeit- oder Reaktionszeitdaten und eine Parametergrenze für das PK-Modell oder das PD-Modell beziehen, wobei der Satz von Daten Parameterwerte umfasst, die einem von PK-Parametern oder PD-Parametern entsprechen (202);
zufälliges Erzeugen, unter Verwendung des HMLCA, eines Satzes von potentiellen Lösungen als eine Population, basierend auf der Parametergrenze und Zuweisen erzeugter Parameterwerte als eine aktuelle beste Formation für jede potentielle Lösung aus dem Satz, wobei jede potentielle Lösung aus der Population Parameterwerte umfasst, die innerhalb der Parametergrenze liegen (204);
Berechnen, unter Verwendung des HMLCA, eines Fitnesswerts für jede potentielle Lösung der Population basierend auf einem oder mehreren Kriterien (206);
Berechnen, unter Verwendung des HMLCA, einer globalen optimalen Lösung unter der Population, wobei die globale optimale Lösung optimale Parameterwerte aus initialisierten Parameterwerten umfasst (208);
Paaren, unter Verwendung des HMLCA, bis ein Stoppkriterium erfüllt ist, der potentiellen Lösungen übereinander basierend auf einer vordefinierten Regel, um eindeutige Paare von potentiellen Lösungen zu erhalten, wobei die eindeutigen Paare von potentiellen Lösungen für (i) einen Vergleich miteinander und (ii) eine anschließende Aktualisierung jeder potentiellen Lösung identifiziert werden (210), und wobei das Stoppkriterium definiert ist, das angibt, wie oft jede Lösung eine Aktualisierung benötigt;
Bestimmen, unter Verwendung des HMLCA, unter Verwendung eines Fitnesswerts, der (i) jeder potentiellen Lösung aus den eindeutigen Paaren von potentiellen Lösungen und (ii) der globalen optimalen Lösung zugeordnet ist, einer potentiellen Lösung vom Typ I und einer potentiellen Lösung vom Typ II aus jedem der eindeutigen Paare (212);
Optimieren, unter Verwendung des HMLCA, des Fitnesswerts der potentiellen Lösung vom Typ I und der potentiellen Lösung vom Typ II durch Durchführen eines Crossovers von einem oder mehreren Parameterwerten, die diesen zugeordnet sind (214);
Erzeugen, unter Verwendung des HMLCA, neuer potentieller Lösungen basierend auf einem aktuellen optimalen Parameterwert der potentiellen Lösung vom Typ I und der potentiellen Lösung vom Typ II (216);
Durchführen, unter Verwendung des HMLCA, eines Vergleichs eines Fitnesswerts der Parameterwerte, die den neuen Lösungen entsprechen, mit einem Fitnesswert von (i) aktuellen optimalen Parameterwerten der neuen Lösungen und (ii) der globalen optimalen Lösung und Aktualisieren, basierend auf dem Vergleich, der aktuellen optimalen Parameterwerte mit den Parameterwerten für jede neue Lösung und die globale optimale Lösung (218);
Eliminieren, unter Verwendung des HMLCA, einer Teilmenge der neuen Lösungen basierend auf einem Vergleich eines Fitnesswerts jeder der neuen Lösungen mit einem Fitnessschwellenwert, um einen gefilterten Satz von Lösungen zu erhalten (220);
Hinzufügen, unter Verwendung des HMLCA, eines neuen Satzes von zufällig erzeugten potentiellen Lösungen in den gefilterten Satz von potentiellen Lösungen, um eine aktualisierte Population zu erhalten (222);
Erzeugen, unter Verwendung des HMLCA, einer globalen optimalen Lösung aus der aktualisierten Population basierend auf einem optimalen Fitnesswert (224); und
Durchführen, unter Verwendung des HMLCA, einer lokalen Optimierungstechnik an der globalen optimalen Lösung, um einen Satz von optimierten Parameterwerten zu schätzen (226).

2. Prozessorimplementiertes Verfahren nach Anspruch 1, wobei jede Lösung, die in dem gefilterten Satz von Lösungen enthalten ist, optimierte Parameterwerte beinhaltet, und wobei jeder der optimierten Parameterwerte einen Fitnesswert umfasst, der kleiner oder gleich dem Fitnessschwellenwert ist.

3. System (100), umfassend:

einen Speicher (102), der Anweisungen speichert;
eine oder mehrere Kommunikationsschnittstellen (106); und
einen oder mehrere Hardwareprozessoren (104), die über die eine oder die mehreren Kommunikationsschnittstellen (106) mit dem Speicher (102) gekoppelt sind, wobei der eine oder die mehreren Hardwareprozessoren (104) durch die Anweisungen konfiguriert sind zum:

EP 3 786 962 B1

Erhalten, unter Verwendung eines Hybrid Modified League Championship Algorithmus, HMLCA, eines Satzes von Daten, die sich auf eines von einem Pharmakokinetik-, PK-, Modell oder einem Pharmakodynamik-, PD-, Modell, Dosierungsinformationen, die dem Satz davon zugeordnet sind, Konzentrationszeit- oder Reaktionszeitdaten und eine Parametergrenze für das PK-Modell oder das PD-Modell beziehen, wobei der Satz von Daten Parameterwerte umfasst, die einem von PK-Parametern oder PD-Parametern entsprechen; zufälliges Erzeugen, unter Verwendung des HMLCA, eines Satzes von potentiellen Lösungen als eine Population basierend auf der Parametergrenze und Zuweisen erzeugter Parameterwerte als aktuelle beste Formation für jede potentielle Lösung aus dem Satz, wobei jede Lösung aus dem Satz von potentiellen Lösungen Parameterwerte umfasst, die innerhalb der Parametergrenze liegen;

Berechnen, unter Verwendung des HMLCA, eines Fitnesswerts für jede potentielle Lösung der Population basierend auf einem oder mehreren Kriterien;

Berechnen, unter Verwendung des HMLCA, einer globalen optimalen Lösung unter der Population, wobei die globale optimale Lösung optimale Parameterwerte aus initialisierten Parameterwerten umfasst;

Paaren, unter Verwendung des HMLCA, bis ein Stoppkriterium erfüllt ist, der potentiellen Lösungen übereinander basierend auf einer vordefinierten Regel, um eindeutige Paare von potentiellen Lösungen zu erhalten, wobei die eindeutigen Paare von potentiellen Lösungen für (i) einen Vergleich miteinander und (ii) eine anschließende Aktualisierung jeder potentiellen Lösung identifiziert werden, und wobei ein Stoppkriterium definiert ist, das angibt, wie oft jede Lösung eine Aktualisierung benötigt;

Bestimmen, unter Verwendung des HMLCA, unter Verwendung eines Fitnesswerts, der (i) jeder potentiellen Lösung aus den eindeutigen Paaren von potentiellen Lösungen und (ii) der globalen optimalen Lösung zugeordnet ist, einer potentiellen Lösung vom Typ I und einer potentiellen Lösung vom Typ II aus jedem der eindeutigen Paare;

Optimieren, unter Verwendung des HMLCA, des Fitnesswerts der potentiellen Lösung vom Typ I und der potentiellen Lösung vom Typ II durch Durchführen eines einheitlichen Crossovers von einem oder mehreren Parameterwerten, die diesen zugeordnet sind;

Erzeugen, unter Verwendung des HMLCA, neuer potentieller Lösungen basierend auf einem aktuellen optimalen Parameterwert der potentiellen Lösung vom Typ I und der potentiellen Lösung vom Typ II;

Durchführen, unter Verwendung des HMLCA, eines Vergleichs eines Fitnesswerts der Parameterwerte, die den neuen Lösungen entsprechen, mit einem Fitnesswert von (i) aktuellen optimalen Parameterwerten der neuen Lösungen und (ii) der globalen optimalen Lösung und Aktualisieren, basierend auf dem Vergleich, der aktuellen optimalen Parameterwerte mit den Parameterwerten für jede neue Lösung und die globale optimale Lösung;

Eliminieren, unter Verwendung des HMLCA, einer Teilmenge der neuen Lösungen basierend auf einem Vergleich eines Fitnesswerts jeder der neuen Lösungen mit einem Fitnessschwellenwert, um einen gefilterten Satz von Lösungen zu erhalten;

Hinzufügen, unter Verwendung des HMLCA, eines neuen Satzes von zufällig erzeugten potentiellen Lösungen in den gefilterten Satz von Lösungen, um eine aktualisierte Population zu erhalten;

Erzeugen, unter Verwendung des HMLCA, einer globalen optimalen potentiellen Lösung aus der aktualisierten Population; und

Durchführen, unter Verwendung des HMLCA, einer lokalen Optimierungstechnik an der optimalen potentiellen Lösung, um einen Satz von optimierten Parameterwerten zu schätzen.

4. System nach Anspruch 3, wobei jede Lösung, die in dem gefilterten Satz von Lösungen enthalten ist, optimierte Parameterwerte beinhaltet, und wobei jeder der optimierten Parameterwerte einen Fitnesswert umfasst, der kleiner oder gleich dem Fitnessschwellenwert ist.

5. Ein oder mehrere nichtflüchtige maschinenlesbare Informationsspeichermedien, die eine oder mehrere Anweisungen umfassen, die bei Ausführung durch einen oder mehrere Hardwareprozessoren die Erzeugung eines optimierten Satzes von pharmakokinetischen, PK, und pharmakodynamischen, PD, Parametern bewirken durch:

Erhalten, unter Verwendung eines Hybrid Modified League Championship Algorithmus, HMLCA, eines Satzes von Daten, die sich auf eines von einem Pharmakokinetik-, PK-, Modell oder einem Pharmakodynamik-, PD-, Modell, Dosierungsinformationen, die dem Satz davon zugeordnet sind, Konzentrationszeit- oder Reaktionszeitdaten und eine Parametergrenze für das PK-Modell oder das PD-Modell beziehen, wobei der Satz von Daten Parameterwerte umfasst, die einem von PK-Parametern oder PD-Parametern entsprechen; zufälliges Erzeugen, unter Verwendung des HMLCA, eines Satzes von potentiellen Lösungen als eine Population basierend auf der Parametergrenze und Zuweisen erzeugter Parameterwerte als eine aktuelle beste Formation für jede potentielle Lösung aus dem Satz, wobei jede der potentiellen Lösungen aus dem Satz

26

Parameterwerte umfasst, die innerhalb der Parametergrenze liegen;

Berechnen, unter Verwendung des HMLCA, eines Fitnesswerts für jede der potentiellen Lösungen basierend auf einem oder mehreren Kriterien, wobei der Fitnesswert in Form einer Zielfunktion berechnet wird, und wobei das eine oder die mehreren Kriterien mindestens eines von Stärke und Schwäche umfassen, die sich auf jede der Mehrzahl von potentiellen Lösungen beziehen;

Berechnen, unter Verwendung des HMLCA, einer globalen optimalen Lösung unter den potentiellen Lösungen/Population, wobei die globale optimale Lösung optimale Parameterwerte aus initialisierten Parameterwerten umfasst;

Paaren, unter Verwendung des HMLCA, bis ein Stoppkriterium erfüllt ist, der potentiellen Lösungen übereinander basierend auf einer vordefinierten Regel, um eindeutige Paare von potentiellen Lösungen zu erhalten, wobei die eindeutigen Paare von potentiellen Lösungen für (i) einen Vergleich miteinander und (ii) eine anschließende Aktualisierung jeder potentiellen Lösung identifiziert werden, und wobei das Stoppkriterium definiert ist, das angibt, wie oft jede Lösung eine Aktualisierung benötigt;

Bestimmen, unter Verwendung des HMLCA, unter Verwendung eines Fitnesswerts, der (i) jeder potentiellen Lösung aus den eindeutigen Paaren von potentiellen Lösungen und (ii) der globalen optimalen Lösung zugeordnet ist, einer potentiellen Lösung vom Typ I und einer potentiellen Lösung vom Typ II aus jedem der eindeutigen Paare;

Optimieren, unter Verwendung des HMLCA, des Fitnesswerts der potentiellen Lösung vom Typ I und der potentiellen Lösung vom Typ II durch Durchführen eines Crossovers von einem oder mehreren Parameterwerten, die diesen zugeordnet sind;

Erzeugen, unter Verwendung des HMLCA, neuer potentieller Lösungen basierend auf dem aktuellen optimalen Parameterwert der potentiellen Lösung vom Typ I und der potentiellen Lösung vom Typ II;

Durchführen, unter Verwendung des HMLCA, eines Vergleichs eines Fitnesswerts der Parameterwerte, die den neuen Lösungen entsprechen, mit einem Fitnesswert von (i) aktuellen optimalen Parameterwerten der neuen Lösungen und (ii) der globalen optimalen Lösung und Aktualisieren, basierend auf dem Vergleich, der aktuellen optimalen Parameterwerte mit den Parameterwerten für jede neue Lösung und die globale optimale Lösung;

Eliminieren, unter Verwendung des HMLCA, einer Teilmenge der neuen Lösungen basierend auf einem Vergleich eines Fitnesswerts jeder der neuen Lösungen mit einem Fitnessschwellenwert, um einen gefilterten Satz von Lösungen zu erhalten, wobei jede Lösung, die in dem gefilterten Satz von Lösungen enthalten ist, optimierte Parameterwerte beinhaltet, und wobei jeder der optimierten Parameterwerte einen Fitnesswert umfasst, der kleiner oder gleich dem Fitnessschwellenwert ist;

Hinzufügen, unter Verwendung des HMLCA, eines neuen Satzes von zufällig erzeugten potentiellen Lösungen in den gefilterten Satz von Lösungen, um eine aktualisierte Population zu erhalten;

Erzeugen, unter Verwendung des HMLCA, einer globalen optimalen Lösung aus der aktualisierten Population basierend auf einem optimalen Fitnesswert; und

Durchführen, unter Verwendung des HMLCA, einer lokalen Optimierungstechnik an der globalen optimalen Lösung, um einen Satz von optimierten Parameterwerten zu schätzen.

6. Ein oder mehrere nichtflüchtige maschinenlesbare Informationsspeichermedien nach Anspruch 5, wobei jede Lösung, die in dem gefilterten Satz von Lösungen enthalten ist, optimierte Parameterwerte beinhaltet.

7. Ein oder mehrere nichtflüchtige maschinenlesbare Informationsspeichermedien nach Anspruch 6, wobei jeder der optimierten Parameterwerte einen Fitnesswert umfasst, der kleiner oder gleich dem Fitnessschwellenwert ist.

## Revendications

1. Procédé mis en œuvre par processeur, comprenant :

l'obtention, par l'intermédiaire d'un ou plusieurs processeurs matériels, à l'aide d'un algorithme de championnat de ligue modifié hybride, HMLCA, d'un ensemble de données se rapportant à l'un d'un modèle de pharmacocinétique, PK, ou d'un modèle de pharmacodynamique, PD, d'informations de dosage associées à l'ensemble de ceux-ci, de données de temps de concentration ou de temps de réponse, et d'une limite de paramètre pour le modèle PK ou le modèle PD, dans lequel l'ensemble de données comprend des valeurs de paramètre correspondant à l'un de paramètres PK ou de paramètres PD (202) ;

la génération aléatoire, à l'aide du HMLCA, d'un ensemble de solutions potentielles en tant que population, sur la base de la limite de paramètre et l'attribution de valeurs de paramètre générées en tant que meilleure formation actuelle pour chaque solution potentielle de l'ensemble, dans lequel chaque solution potentielle de la population comprend des valeurs de paramètre qui sont dans la limite de paramètre (204) ;

le calcul, à l'aide du HMLCA, d'une valeur d'aptitude pour chaque solution potentielle de la population sur la base d'un ou plusieurs critères (206) ;

le calcul, à l'aide du HMLCA, d'une solution optimale globale parmi la population, dans lequel la solution optimale globale comprend des valeurs de paramètre optimales à partir de valeurs de paramètre initialisées (208) ;

l'appariement, à l'aide du HMLCA, jusqu'à ce qu'un critère d'arrêt soit satisfait, des solutions potentielles les unes aux autres sur la base d'une règle prédéfinie pour obtenir des paires uniques de solutions potentielles, dans lequel les paires uniques de solutions potentielles sont identifiées pour (i) une comparaison les unes par rapport aux autres et (ii) une mise à jour ultérieure de chaque solution potentielle (210), et dans lequel le critère d'arrêt est défini qui est indicatif du nombre de fois que chaque solution a besoin d'une mise à jour ;

la détermination, à l'aide du HMLCA, à l'aide d'une valeur d'aptitude associée à (i) chaque solution potentielle des paires uniques de solutions potentielles et (ii) la solution optimale globale, d'une solution potentielle de type I et d'une solution potentielle de type II de chacune des paires uniques (212) ;

l'optimisation, à l'aide du HMLCA, de la valeur d'aptitude de la solution potentielle de type I et de la solution potentielle de type II en effectuant un croisement d'une ou plusieurs valeurs de paramètre associées à celles-ci (214) ;

la génération, à l'aide du HMLCA, de nouvelles solutions potentielles sur la base d'une valeur de paramètre optimale actuelle de la solution potentielle de type I et de la solution potentielle de type II (216) ;

l'exécution, à l'aide du HMLCA, d'une comparaison d'une valeur d'aptitude des valeurs de paramètre correspondant aux nouvelles solutions avec une valeur d'aptitude de (i) valeurs de paramètre optimales actuelles des nouvelles solutions et (ii) la solution optimale globale et la mise à jour, sur la base de la comparaison, des valeurs de paramètre optimales actuelles avec les valeurs de paramètre pour chaque nouvelle solution et la solution optimale globale (218) ;

l'élimination, à l'aide du HMLCA, d'un sous-ensemble des nouvelles solutions sur la base d'une comparaison d'une valeur d'aptitude de chacune des nouvelles solutions avec un seuil d'aptitude pour obtenir un ensemble filtré de solutions (220) ;

l'ajout, à l'aide du HMLCA, d'un nouvel ensemble de solutions potentielles générées de manière aléatoire dans l'ensemble filtré de solutions potentielles pour obtenir une population mise à jour (222) ;

la génération, à l'aide du HMLCA, d'une solution optimale globale à partir de la population mise à jour sur la base d'une valeur d'aptitude optimale (224) ; et

l'exécution, à l'aide du HMLCA, d'une technique d'optimisation locale sur la solution optimale globale pour estimer un ensemble de valeurs de paramètre optimisées (226).

2. Procédé mis en œuvre par processeur selon la revendication 1, dans lequel chaque solution comprise dans l'ensemble filtré de solutions comprend des valeurs de paramètre optimisées, et dans lequel chacune des valeurs de paramètre optimisées comprend une valeur d'aptitude qui est inférieure ou égale au seuil d'aptitude.

3. Système (100) comprenant :

une mémoire (102) stockant des instructions ;
une ou plusieurs interfaces de communication (106) ; et
un ou plusieurs processeurs matériels (104) couplés à la mémoire (102) via les une ou plusieurs interfaces de communication (106), dans lequel les un ou plusieurs processeurs matériels (104) sont configurés par les instructions pour :

obtenir, à l'aide d'un algorithme de championnat de ligue modifié hybride, HMLCA, un ensemble de données se rapportant à l'un d'un modèle de pharmacocinétique, PK, ou d'un modèle de pharmacodynamique, PD, d'informations de dosage associées à l'ensemble de ceux-ci, de données de temps de concentration ou de temps de réponse, et d'une limite de paramètre pour le modèle PK ou le modèle PD, dans lequel l'ensemble de données comprend des valeurs de paramètre correspondant à l'un de paramètres PK ou de paramètres PD ;

générer de manière aléatoire, à l'aide du HMLCA, un ensemble de solutions potentielles en tant que population sur la base de la limite de paramètre et attribuer des valeurs de paramètre générées en tant que meilleure formation actuelle pour chaque solution potentielle de l'ensemble, dans lequel chaque solution de l'ensemble de solutions potentielles comprend des valeurs de paramètre qui sont dans la limite de paramètre;

calculer, à l'aide du HMLCA, une valeur d'aptitude pour chaque solution potentielle de la population sur la base d'un ou plusieurs critères ;

calculer, à l'aide du HMLCA, une solution optimale globale parmi la population, dans lequel la solution optimale globale comprend des valeurs de paramètre optimales à partir de valeurs de paramètre initialisées ;

apparier, à l'aide du HMLCA, jusqu'à ce qu'un critère d'arrêt soit satisfait, les solutions potentielles les unes aux autres sur la base d'une règle prédéfinie pour obtenir des paires uniques de solutions potentielles, dans lequel les paires uniques de solutions potentielles sont identifiées pour (i) une comparaison les unes par rapport aux autres et (ii) une mise à jour ultérieure de chaque solution potentielle, et dans lequel un critère d'arrêt est défini qui est indicatif du nombre de fois que chaque solution a besoin d'une mise à jour ;

déterminer, à l'aide du HMLCA, à l'aide d'une valeur d'aptitude associée à (i) chaque solution potentielle des paires uniques de solutions potentielles et (ii) la solution optimale globale, une solution potentielle de type I et une solution potentielle de type II de chacune des paires uniques ;

optimiser, à l'aide du HMLCA, la valeur d'aptitude de la solution potentielle de type I et de la solution potentielle de type II en effectuant un croisement uniforme d'une ou plusieurs valeurs de paramètre associées à celles-ci ;

générer, à l'aide du HMLCA, de nouvelles solutions potentielles sur la base d'une valeur de paramètre optimale actuelle de la solution potentielle de type I et de la solution potentielle de type II ;

effectuer, à l'aide du HMLCA, une comparaison d'une valeur d'aptitude des valeurs de paramètre correspondant aux nouvelles solutions avec une valeur d'aptitude de (i) valeurs de paramètre optimales actuelles des nouvelles solutions et (ii) la solution optimale globale et mettre à jour, sur la base de la comparaison, les valeurs de paramètre optimales actuelles avec les valeurs de paramètre pour chaque nouvelle solution et la solution optimale globale ;

éliminer, à l'aide du HMLCA, un sous-ensemble des nouvelles solutions sur la base d'une comparaison d'une valeur d'aptitude de chacune des nouvelles solutions avec un seuil d'aptitude pour obtenir un ensemble filtré de solutions ;

ajouter, à l'aide du HMLCA, un nouvel ensemble de solutions potentielles générées de manière aléatoire dans l'ensemble filtré de solutions pour obtenir une population mise à jour ;

générer, à l'aide du HMLCA, une solution potentielle optimale globale à partir de la population mise à jour ; et

effectuer, à l'aide du HMLCA, une technique d'optimisation locale sur la solution potentielle optimale pour estimer un ensemble de valeurs de paramètre optimisées.

4. Système selon la revendication 3, dans lequel chaque solution comprise dans l'ensemble filtré de solutions comprend des valeurs de paramètre optimisées, et dans lequel chacune des valeurs de paramètre optimisées comprend une valeur d'aptitude qui est inférieure ou égale au seuil d'aptitude.

5. Un ou plusieurs supports de stockage d'informations lisibles par machine non transitoires comprenant une ou plusieurs instructions qui, lorsqu'elles sont exécutées par un ou plusieurs processeurs matériels, provoquent la génération d'un ensemble optimisé de paramètres de pharmacocinétique, PK, et de pharmacodynamique, PD, par :

l'obtention, à l'aide d'un algorithme de championnat de ligue modifié hybride, HMLCA, d'un ensemble de données se rapportant à l'un d'un modèle de pharmacocinétique, PK, ou d'un modèle de pharmacodynamique, PD, d'informations de dosage associées à l'ensemble de ceux-ci, de données de temps de concentration ou de temps de réponse, et d'une limite de paramètre pour le modèle PK ou le modèle PD, dans lequel l'ensemble de données comprend des valeurs de paramètre correspondant à l'un de paramètres PK ou de paramètres PD ;

la génération aléatoire, à l'aide du HMLCA, d'un ensemble de solutions potentielles en tant que population sur la base de la limite de paramètre et l'attribution de valeurs de paramètre générées en tant que meilleure formation actuelle pour chaque solution potentielle de l'ensemble, dans lequel chacune des solutions potentielles de l'ensemble comprend des valeurs de paramètre qui sont dans la limite de paramètre;

le calcul, à l'aide du HMLCA, d'une valeur d'aptitude pour chacune des solutions potentielles sur la base d'un ou plusieurs critères, dans lequel la valeur d'aptitude est calculée sous la forme d'une fonction objective, et dans lequel les un ou plusieurs critères comprennent au moins l'une d'une force et d'une faiblesse se rapportant à chacune de la pluralité de solutions potentielles ;

le calcul, à l'aide du HMLCA, d'une solution optimale globale parmi les solutions/population potentielles, dans lequel la solution optimale globale comprend des valeurs de paramètre optimales à partir de valeurs de paramètre initialisées ;

l'appariement, à l'aide du HMLCA, jusqu'à ce qu'un critère d'arrêt soit satisfait, des solutions potentielles les unes aux autres sur la base d'une règle prédéfinie pour obtenir des paires uniques de solutions potentielles, dans lequel les paires uniques de solutions potentielles sont identifiées pour (i) une comparaison les unes par rapport aux autres et (ii) une mise à jour ultérieure de chaque solution potentielle, et dans lequel le critère d'arrêt est défini qui est indicatif du nombre de fois que chaque solution a besoin d'une mise à jour ;

la détermination, à l'aide du HMLCA, à l'aide d'une valeur d'aptitude associée à (i) chaque solution potentielle des paires uniques de solutions potentielles et (ii) la solution optimale globale, une solution potentielle de type I et une solution potentielle de type II de chacune des paires uniques ;

l'optimisation, à l'aide du HMLCA, de la valeur d'aptitude de la solution potentielle de type I et de la solution potentielle de type II en effectuant un croisement d'une ou plusieurs valeurs de paramètre associées à celles-ci ;

la génération, à l'aide du HMLCA, de nouvelles solutions potentielles sur la base de la valeur de paramètre optimale actuelle de la solution potentielle de type I et de la solution potentielle de type II ;

l'exécution, à l'aide du HMLCA, d'une comparaison d'une valeur d'aptitude des valeurs de paramètre correspondant aux nouvelles solutions avec une valeur d'aptitude de (i) valeurs de paramètre optimales actuelles des nouvelles solutions et (ii) la solution optimale globale et la mise à jour, sur la base de la comparaison, des valeurs de paramètre optimales actuelles avec les valeurs de paramètre pour chaque nouvelle solution et la solution optimale globale ;

l'élimination, à l'aide du HMLCA, d'un sous-ensemble des nouvelles solutions sur la base d'une comparaison d'une valeur d'aptitude de chacune des nouvelles solutions avec un seuil d'aptitude pour obtenir un ensemble filtré de solutions, dans lequel chaque solution comprise dans l'ensemble filtré de solutions comprend des valeurs de paramètre optimisées, et dans lequel chacune des valeurs de paramètre optimisées comprend une valeur d'aptitude qui est inférieure ou égale au seuil d'aptitude ;

l'ajout, à l'aide du HMLCA, d'un nouvel ensemble de solutions potentielles générées de manière aléatoire dans l'ensemble filtré de solutions pour obtenir une population mise à jour ;

la génération, à l'aide du HMLCA, d'une solution optimale globale à partir de la population mise à jour sur la base d'une valeur d'aptitude optimale ; et

l'exécution, à l'aide du HMLCA, d'une technique d'optimisation locale sur la solution optimale globale pour estimer un ensemble de valeurs de paramètre optimisées.

6. Un ou plusieurs supports de stockage d'informations lisibles par machine non transitoires selon la revendication 5, dans lequel chaque solution comprise dans l'ensemble filtré de solutions comprend des valeurs de paramètre optimisées.

7. Un ou plusieurs supports de stockage d'informations lisibles par machine non transitoires selon la revendication 6, dans lequel chacune des valeurs de paramètre optimisées comprend une valeur d'aptitude qui est inférieure ou égale au seuil d'aptitude.

**FIG. 1 (PRIOR ART)**

SYSTEM
100

MEMORY
102

108

HARDWARE
PROCESSOR(S)
104

INTERFACE(S)
106

**FIG. 2**

obtaining a set of data pertaining to one of a Pharmacokinetics (PK) model or a Pharmacodynamics (PD) model, dosage information associated with the set thereof, concentration-time or a response-time data, and a parameter boundary for the PK model or PD model, wherein the set comprises parameters values corresponding to one of PK parameters or PD parameters ⌇~ 202

randomly generating potential solutions as a population, based on the parameter boundary and assigning generated parameter values as a current best formation for each potential solution from the set, wherein each solution comprises unique parameter values that are within the parameter boundary ⌇~ 204

computing a fitness value for each of the potential solutions based on one or more criteria ⌇~ 206

computing a global optimal solution among the population, wherein the global optimal solution comprises optimal parameter values from initialized parameter values ⌇~ 208

pairing, until a stopping criteria is satisfied, the potential solutions across each other based on a predefined rule to obtain unique pairs of potential solutions, the unique pairs of potential solutions are being identified for (i) comparison against each other and (ii) subsequent updation of each potential solution, the stopping criteria is defined that is indicative of number of times each solution needs an update ⌇~ 210

determining, using a fitness value associated with (i) each potential solution from the unique pairs of potential solutions and (ii) the global optimal solution, a type I potential solution and a type II potential solution from each of the unique pairs ⌇~ 212

(A)

**FIG. 3A**

A

optimizing the fitness value of the type I potential solution and type II potential solution by performing a crossover of one or more parameter values associated thereof — 214

generating new potential solutions based on a current optimal parameter value of the type I potential solution and type II potential solution — 216

performing a comparison of a fitness value of the parameter values corresponding to the new solutions with a fitness value of (i) current optimal parameter values of the new solutions and (ii) the global optimal solution and updating, based on the comparison, the current optimal parameter values with the parameter values for each new solution and the global optimal solution — 218

eliminating a subset of the new solutions based on a comparison of a fitness value of each of the new solutions with a fitness threshold to obtain a filtered set of solutions — 220

adding a new set of randomly generated potential solutions into the filtered set of potential solutions to obtain an updated population — 222

generating a global optimal solution from the updated population based on an optimal fitness value — 224

performing a local optimization technique on the global optimal solution to estimate a set of optimized parameter values — 226

FIG. 3B

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- IN 201921029215
- US 2015269225 A1
- EP 3422358 A1
- KR 20110119409 A
- WO 2016000035 A1
- US 2003088320 A1

### Non-patent literature cited in the description

- League championship algorithm: a new algorithm for numerical function optimization. **KASHAN** ; **ALI** ; **HUSSEINZADEH**. 2009 International Conference of Soft Computing and Pattern Recognition. IEEE, 2009
- **GABRIELSSON, JOHAN** ; **DANIEL WEINER**. Pharmacokinetic and pharmacodynamic data analysis: concepts and applications. CRC Press, 2006